# EUROPEAN PATENT APPLICATION

(11) **EP 1 225 222 A1**
(43) Date of publication of application: **24.07.2002**
(21) Application number: 00946355.5
(22) Date of filing: 14.07.2000
(51) Int. Cl.: C12N 15/11, C07K 14/82, C07K 16/32, A61K 31/711, A61K 48/00, A61K 35/00

(54) **NOVEL TUMOR SUPPRESSOR GENE**

(30) Priority: 29.10.1999 JP 31042099
(71) Applicant: MOCHIDA PHARMACEUTICAL CO., LTD., Shinjuku-ku Tokyo 160-8515 (JP)
(72) Inventor: KOMINAMI, Ryo, Niigata-shi, Niigata 950-2045 (JP)
(74) Representative: Grünecker, Kinkeldey, Stockmair & Schwanhäusser Anwaltssozietät
(86) International application number: JP0004765
(87) International publication number: WO0132859

(57) **Abstract**

A novel tumor suppressor gene and a novel polypeptide having a 2-3 type size zinc finger structure which is an amplification product of the tumor suppressor gene. The above gene and polypeptide are usable in diagnosing cancer, estimating the risk of carcinogenesis and gene therapy.

## Description

### TECHNICAL FIELD

This invention relates to a novel gene and a novel polypeptide which is the product of such novel gene. This invention also relates to an antibody which recognizes said novel polypeptide, and a drug screening method and a cancer diagnosing method which utilize any one of such gene, polypeptide, and antibody of the present invention.

### BACKGROUND ART

Ever since the cloning of Rb gene as an anti-oncogene (Friend, SH. et al, Nature 323, 643-646, 1986), the mechanism of cell transformation has been analyzed from the vantage point of oncogene and anti-oncogene (tumor suppressor gene) expression.

Since a tumor suppressor gene is often mapped in a cancer cell in the chromosomal region where loss of one or both alleles is found at a high frequency, it has been commonplace to conduct LOH (loss of heterozygosity) analysis in the identification of a tumor suppressing gene. As a typical site in a cancer cell where LOH is found, Hegi, for example, has indicated the terminal region of chromosome 4 (corresponding to lp36 in human) as the site of allelic loss after analyzing a lung tumor cell (Hegi ME, et al., Cancer Res., 54, 6257-6264, 1994). Such allelic loss is also found in breast cancer cell, and an analysis using mouse thymic lymphoma has narrowed this site to the range as narrow as 0.6 cM (centimorgan) (Santos J. et al., Oncogene 12, 669-676, 1996; Santos J. et al., Oncogene 17, 925-929, 1998). With regard to human cancer, allelic loss has been reported in 14q32 region of chromosome 14 in ovarian cancer cell and neuroblastoma cell (Suzuki T., Cancer Res., 49, 1095-1098, 1989; Chang WY-H et al, Cancer Res., 55, 3246-3249, 1995). In order to identify the tumor suppressor gene, further narrowing of the site where LOH has been found and finally, identification to the level of the gene is required. Microsatellite markers, STSs, ESTs, and various other markers that had been reported along with the progress of Human Genome Project have been useful means in the mapping of the tumor suppressor gene. The information provided, however, has been predominantly the sequence information and not much of the functional information, and identification of tumor suppressor gene is still facing with difficulties and the tumor suppressor gene is not identified in the case of the aforementioned tumor suppressor gene on the chromosome 14. A diverse tumor suppressor genes are believed to be functioning in a body depending on the type of animal, the type of cancer, and the type of cell. There has so far been identified only about 25 tumor suppressor genes, and this number is by far too small compared to the number of cancer cell types.

### DISCLOSURE OF THE INVENTION

In view of preventing cancer, improvement in the technology of diagnosing cell transformation and determining the cancer risk is important, and identification of new tumor suppressor genes is required for such improvement. The inventors of the present invention have made an intensive study to identify new genes associated with the cell transformation, and by focusing on γ ray-induced mouse lymphoma, the inventors found that LOH is occurring in the region of about 2.9 cM on chromosome 12 after conducing LOH analysis using such lymphoma. To be more specific, the inventors made markers required in the mapping to enable detailed analysis, and succeeded in narrowing the LOH-occurring region to the region of about 35 kb. When this region was partly analyzed for its nucleotide sequence, it was found that Not I restriction enzyme site is included in this region, that 16 mouse ESTs of 4 different types are present in this region, and that one of such EST showed homology with human EST on q32 on chromosome 14 confirming that this region is the region located on 14q32. Although it might be possible to determine the probability of cell transformation by confirming the occurrence of the mutation in this chromosomal region, an accurate diagnosis would be enabled only after the identification of the tumor suppressor gene in this region. The inventors of the present invention have conducted an analysis by producing further markers in order to identify the tumor suppressor gene, and thereby identified one candidate gene. It was then confirmed that this gene was not normally expressed in the case of lymphoma to arrive at the conclusion that this gene was the novel tumor suppressor gene.

In view of the situation as described above, this invention provides the novel tumor suppressor gene as described above and an oligonucleotide which is a fragment of such tumor suppressor gene. Also provided are a novel polypeptide and a fragment thereof translated from such gene.

The present invention also provides a vector containing the novel tumor suppressor gene of the present invention, and a transformant produced by transforming with such vector.

The present invention also provides an antibody produced by administering said novel polypeptide or the polypeptide fragment which is a fragment of said novel polypeptide as an antigen.

The present invention also provides a method for diagnosing cancer or cancer risk wherein mutation or polymorphism of said novel tumor suppressor gene, or mutation or polymorphism of the region including said novel tumor suppressor gene on the chromosome is used as an index. Also provided is a method for applying said novel tumor suppressor gene to gene therapy.

### BRIEF DESCRIPTION OF THE DRAWINGS

FIG. 1 shows a schematic structural view of the novel protein of the present invention, and schematic views of its isoforms. The numbers in the drawing designate the number of exons.
FIG. 2 is a view showing the result of the multiplex PCR in Example 3(1).
FIG. 3 is a view showing the result of the RT-PCR in Example 3(3).

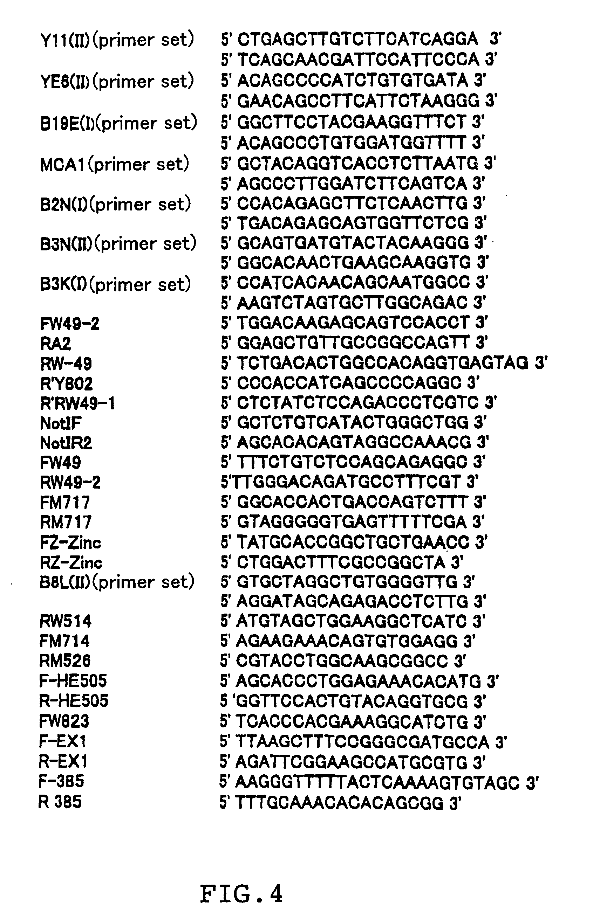

FIG. 4 is a view showing the nucleotide sequence of the primer used in the Examples.

### BEST MODE FOR CARRYING OUT THE INVENTION

The present invention is hereinafter described in detail.

This invention relates to a tumor suppressor gene. This invention also relates to a novel polypeptide coded by such tumor suppressor gene. The novel polypeptide of the present invention is characterized by having a zinc finger structure of 2-3 type with 6 C₂H₂ zinc finger motifs. C₂H₂ zinc finger is a DNA-binding structure which is found in a transcription factor, and in the C2H2 zinc finger, 2 Cys and 2 His are separated by a loop of 12 amino acid residues with the basic structure being Cys - X_{(2 to 4)} - Cys - X₍₃₎ - Phe - X₍₅₎ - Leu - X₍₂₎ - His - X_{(3 to 5)} - His - X (wherein X can be any amino acid; number in the brackets indicate the number of amino acid residues; and Phe and Leu may be other amino acids). When expressed in one letter abbreviation of the amino acids, the C₂H₂ zinc finger may be expressed as C- X_{(2 to 4)} - C - X(₍₃₎ - F - X₍₅₎ - L -X₍₂₎ - H - X_{(3 to 5)} - H - X. The 6 zinc fingers in the novel polypeptide of the present invention are separated in 3 clusters. The cluster located in on the side nearest to the N terminal comprises 1 zinc finger, and the cluster located on the side nearest to the C terminal comprises 3 zinc fingers, and the cluster in the middle comprises as 2 zinc fingers. Such polypeptide including the cluster comprising 2 zinc fingers and the cluster comprising 3 zinc fingers, and wherein another cluster comprising 2 zinc finger is located on the side of the N terminal is designated in the present specification as "a polypeptide having a zinc finger structure of 2-3 type". The polypeptide having such 2-3 zinc finger structure has been unknown for the C₂H₂ zinc finger, and this is a polypeptide of a new category. It is estimated that the zinc finger structure of 2-3 type in such polypeptide is associated with the specific binding of the polypeptide to the genes controlling proliferation/differentiation or apoptosis of the cell in cells like thymus cell, lymphocyte, neuron, and meninges cell.

The novel polypeptide of the present invention is not limited for its origin or its sequence as long as it has the C2H2 zinc finger domain of 2-3 type. The preferable examples of the polypeptide of the present invention, however, are those having any one of the zinc finger motifs shown in Table 1 (Sequence Listing, SEQ ID NO: 2) or Table 2 (Sequence Listing, SEQ ID NO: 4).

Examples of the polypeptides which are more preferable in the present invention are those having the function of suppressing the cell transformation, for example, the polypeptides having the amino acid sequence of amino acid Nos. 1 to 884 or the amino acid sequence of amino acid Nos. 47 to 884 in Table 1, and also, the polypeptide having the amino acid sequence of amino acid Nos. 1 to 894 or the amino acid sequence of amino acid Nos. 47 to 894 in Table 2. Such amino acid sequences shown in Tables 1 and 2 are the amino acid sequences of the polypeptides from murine cell and human cell, respectively. The polypeptide having the function of suppressing the cell transformation plays important roles in organism, and the amino acid sequence of such biologically important polypeptide is often conserved beyond the species, and it is estimated that the novel polypeptide of the present invention is also found in the animals other than mouse and human as a polypeptide having a high homology with an amino acid sequence shown in Table 1 or 2, and in particular, that the amino acid sequence of the part of the zinc finger shown in Table 1 or 2. Accordingly, examples of the preferable novel polypeptide of the present invention include polypeptides exhibiting a homology of 80% or higher, and preferably 90% or higher with amino acid Nos. 1 to 884 or 47 to 884 in the amino acid sequence shown in Table 1, or amino acid Nos. 1 to 894 or 47 to 894 in the amino acid sequence shown in Table 2. Also included in the examples of the preferable novel polypeptide of the present invention are polypeptide having 2-3 zinc finger structure including as its sequence of the zinc finger portion the sequence exhibiting a homology of 80% or higher, and preferably 90% or higher with any one of the zinc fingers shown in amino acid Nos. 223 to 244, 428 to 448, 456 to 476, 788 to 808, 816 to 836, and 846 to 867 in Table 1, or in amino acid Nos. 223 to 244, 429 to 449, 457 to 477, 798 to 817, 826 to 846, and 856 to 877 of Table 2.

By the way, those skilled in the art can readily carry out site-directed mutagenesis or the like to thereby replace, delete, add, or insert at least one amino acid of the polypeptide with no influence on the basic activity of the polypeptide. Therefore, also included within the scope of the present invention are polypeptides having the amino acid sequence wherein replacement, deletion, addition, or insertion has occurred in at least one amino acid in the amino acid sequences of Table 1 and 2.

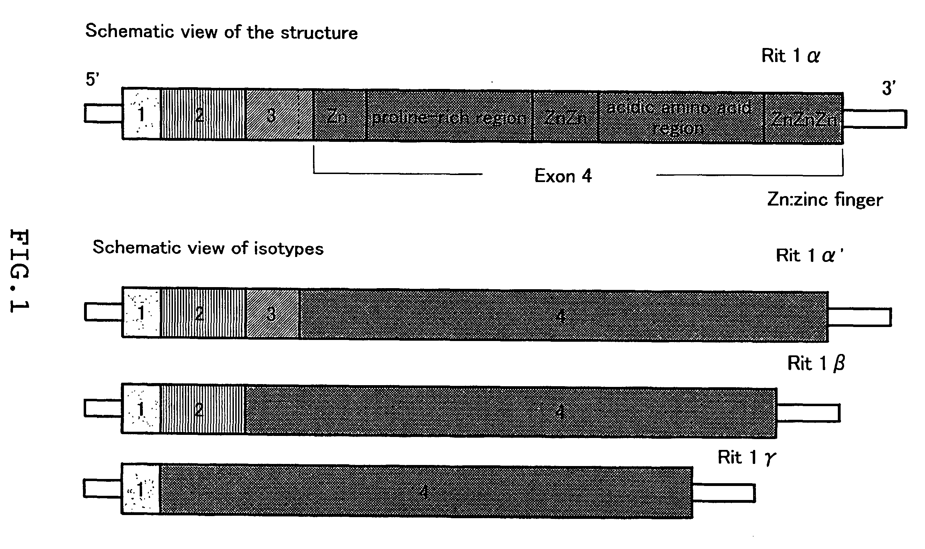

As shown in FIG. 1, the polypeptides found in the organisms include the polypeptide including all of the exons 1, 2, 3, and 4 of Table 3 or 4 (Rit1α in FIG. 1), the polypeptide wherein the exons 2 and 3 are missing (Rit1γ in FIG. 1), the polypeptide wherein the exon 3 is missing (Rit1β in FIG. 1), and the polypeptide wherein a part of the exon 3 is missing (Rit1α' in FIG. 1) (SEQ ID NO: 5, 6, 7, 8, 9, 10, 11, 12, 13, 14, 15, and 16 in the Sequence Listing). It is estimated that these isoforms are expressed in different parts of the body. The polypeptide of the present invention includes such isoforms as well as the polypeptide having the sequence wherein the exons 1 to 4 are combined in an arbitrary manner.

This invention also provides an arbitrary fragment of the novel polypeptide as described above. The polypeptide fragment may be any part of the above-described novel polypeptide, and may comprise any number of the above-described amino acid sequences. The polypeptide fragment, however, is preferably a fragment comprising or including the amino acid sequences corresponding to the exons shown in Table 5 or 6, or alternatively, a fragment containing the amino acid sequence of at least one region selected from the zinc fingers, the zinc finger clusters (the regions shown as amino acid Nos. 428 to 476 and 788 to 867 in Table 1, and amino acid Nos. 429 to 477 and 798 to 877 in Table 2), the proline-rich region, and the acidic amino acid region shown in Tables 1 and 2. The polypeptide fragment which is a part of the novel polypeptide of the present invention can be used as an antigen which is administered in producing an antibody which specifically recognizes the novel polypeptide of the present invention. When the polypeptide fragment is used for such an antigen to be administered, the polypeptide fragment may preferably contain the part comprising at least six amino acids.

The novel polypeptide of the present invention and the fragment thereof may be produced by a genetic engineering means. The vector and the transformant which may be used in producing the novel polypeptide of the present invention by a genetic engineering means will be described later in the sections directed to the vector and the transformant of the present invention. In producing the novel polypeptide of the present invention, the transformant of the present invention is first cultivated, and if necessary, gene amplification and induction of polypeptide expression are conducted. The cultivation of the transformant and the induction of the polypeptide expression may be carried out in accordance with a known method (see for example, "Experimental Procedures in Microbiology", edited by Corporation Aggregate Japan Biochemistry Society, Tokyo Kagaku Dojin K.K., 1992). Next, the transformant is recovered, and the desired polypeptide may be purified as desired from the recovered cell by combining appropriate procedures such as concentration, solubilization, dialysis, and various chromatographic processes. Purification, however, may be accomplished at a high efficiency when the step using the affinity column wherein the antibody of the present invention as will be described later is adsorbed on an agarose gel is included as one purification step.

Next, the novel gene of the present invention is described. The novel gene of the present invention has a characteristic feature that it codes for a polypeptide having a C2H2 zinc finger of 2-3 type. The novel gene is preferably the gene which codes for a polypeptide having the function of controlling the proliferation/differentiation or apoptosis of the cell, and more preferably, the gene coding for a polypeptide having the function of suppressing the cell transformation. The novel gene of the present invention is not limited for its origin and it may be a gene from any animal, and also, the novel gene of the present invention may be a genomic DNA, a cDNA, or a mRNA. The gene of the present invention may also be a sense strand or an antisense strand, and may be in the form of a single strand or a double strand.

The preferable examples of the cDNA of the present invention are those having the nucleotide sequence coding for the amino acid sequence shown in Table 1 or 2, and more preferably, those having the nucleotide sequence shown in Table 3 (Sequence Listing, SEQ ID NO: 1) or Table 4 (Sequence Listing, SEQ ID NO: 3). These correspond to the cDNA of the novel tumor suppressor gene obtained from mouse and human, respectively. The cDNA of the present invention are more preferably those having the nucleotide sequence shown in nucleotide Nos. 272 to 2923 or 410 to 2923 in Table 3, or nucleotide Nos. 11 to 2692 or 149 to 2692 in Table 4. It is believed that the preferable nucleotide sequences as mentioned above are those which are the coding region in mouse and human, respectively. When degeneracy of codon is taken into consideration, a nucleotide sequence can be modified by replacing a part of the sequence with other nucleotide(s) with no change in the amino acid coded by the nucleotide sequence, and therefore, the sequences wherein one or more nucleotide is replaced with another nucleotide in the nucleotide sequences shown in Table 3 or 4 are also included within the scope of the cDNA of the present invention as long as it codes for the amino acid sequence of Table 1 or 2.

As will be shown in the Examples, there have been confirmed in a mouse cell an isoform wherein the region corresponding to the exon 3 of Table 3 has spliced out, an isoform wherein a part (12 nucleotides) at the 3' end of the exon 3 in Table has spliced out, and wherein the region corresponding to the exons 2 and 3 has spliced out. Accordingly, other preferable examples of the cDNA of the present invention include those having the nucleotide sequence coding for such an isoform (Sequence Listing, SEQ ID NO: 5, 7, 9, 11, 13, and 15), and those wherein the sequences of the exon regions shown in Table 3 or 4 are combined in an arbitrary order.

As mentioned above, it is highly likely that the tumor suppressor gene is conserved across species, and accordingly, also included within the scope of the cDNA of the present invention are those having a homology of 80% or higher, and preferably, a homology of 90% or higher with the nucleotide sequence of nucleotide Nos. 410 to 2923 in Table 3 or the nucleotide sequence of nucleotide Nos. 149 to 2692 in Table 4. Preferable examples of the cDNA of the present invention include those having the nucleotide sequence with a homology of 80% or higher, and preferably, a homology of 90% or higher with the nucleotide sequence of nucleotide Nos. 938 to 1003, 1553 to 1615, 1637 to 1699, 2633 to 2695, 2717 to 2779, or 2807 to 2872 in Table 3, or the nucleotide sequence of nucleotide Nos. 677 to 742, 1295 to 1357, 1379 to 1441, 2402 to 2464, 2486 to 2548, or 2576 to 2641 in Table 4 as the nucleotide sequence coding for the zinc finger region.

In the meanwhile, preferable examples for the mRNA of the present invention are the sequences wherein thymidine (T) is replaced with uracil (U) in the nucleotide sequences of the above-described cDNA and its preferable examples.

The genomic DNA is the region on the chromosome which is necessary for the expression of the polypeptide of the present invention, and included in the scope of the present invention are those including transcription control regions in addition to the exon and the intron.

Preferable examples of the genomic DNA are those containing the nucleotide sequence of exons 1 to 4 of Table 3 or 4 as the exon or a part thereof. It is to be noted that the starting position is shown in the table for every parts corresponding to the exons.

As described above, it is likely that the gene is conserved across the species, and a high homology is expected for the region of the exons, and in particular, the fourth exon coding for the zinc finger structure. Therefore, a genomic DNA wherein the exon region in the gene has a sequence exhibiting a homology of 80% or higher, and more preferably, a homology of 90% or more with any one of the exon region shown in Table 3 or 4 is within the scope of the genomic DNA of the present invention. Among such genomic DNA, genomic DNAs wherein the region of the exon including the zinc finger has a homology of 80% or higher, and preferably, 90% or higher with the region of the fourth region in Table 3 or 4 are preferable exmaples of the genomic DNA of the present invention.

This invention also provides a DNA or an RNA fragment which contains an arbitrary region of the cDNA, the genomic DNA, or the mRNA of the present invention. The fragment of the cDNA, the genomic DNA, or the mRNA of the present invention may be a double stranded fragment, an arbitrary part of the sense strand, or an arbitrary part or the antisense strand. Although the DNA or the RNA fragment is not particularly limited for its length and sequence, the DNA or the RNA fragment is preferably a fragment comprising 13 or more nucleotides, and more preferably, 15 or more nucleotides. Preferable examples of the DNA or the RNA fragment of the present invention are a DNA fragments having a nucleotide sequence which corresponds to an exon region in Table 3 or 4, or an RNA fragment corresponding to such DNA fragment, a DNA fragment having a nucleotide sequence of the zinc finger region in Table 3 or 4, or an RNA fragment corresponding to such DNA fragment, a DNA fragment having a nucleotide sequence of the proline-rich region or the acidic amino acid region in Table 3 or 4.

The gene, the DNA fragment, and the RNA fragment of the present invention may be the one wherein a part or all of the nucleotides constituting the strand are artificially synthesized derivatives. The DNA fragment and the RNA fragment of the present invention may also be the one which has been labeled with a radio isotope, a fluorescent substance, an enzyme, or a luminescent substance as desired. As will be described later, such DNA fragment or RNA fragment can be used as a probe or a PCR primer in the diagnosis of cancer or cancer risk wherein normal expression of the tumor suppressor gene of the present invention in a cell is used for the index.

Next, a typical method for obtaining the novel cDNA, genomic DNA, and mRNA of the present invention is described.

The cDNA, genomic DNA, and mRNA of the present invention may be produced by means of chemical synthesis using a known method, or by PCR amplification from the nucleotide sequence of the desired part. When the cDNA of the present invention is obtained by PCR amplification, an adequate part is selected from the nucleotide sequence shown in Table 3 or 4, and several sets of primers coding for the selected sequence each comprising at least 15 nucleotides are chemically synthesized by a known method. Next, PCR is conducted according to a known method using a cDNA library from an appropriate organism for the template (see, for example, Michael, A.I. et al., PCR Protocols, a guide to method and application, Academic Press, 1990). Alternatively, the DNA of the present invention may be obtained by preparing a probe on the bases of the nucleotide sequence shown in Table 3 or 4, and conducting the hybridization under highly stringent conditions. Although the cDNA library used is not limited for the species of the source organism, the source cell is preferably a normal cell or a normal tissue. The resulting amplification product is analyzed for its nucleotide sequence, and based on the results of such analysis, ligation may be conducted using a DNA ligase or the like so that the cDNA covers the entire region of the cDNA to be translated to thereby obtain the full length cDNA. Similarly, the genomic DNA can be obtained by PCR using the DNA extracted from an appropriate organism or by using a genomic DNA library from an appropriate organism, or by hybridization. A full length genomic DNA (intact gene) can be obtained by ligating the resulting genomic DNA fragments. This process can be efficiently accomplished when primers are prepared by utilizing the nucleotide sequence of the part of the exon shown in Table 3 or 4. The mRNA can also be produced by means of PCR from the mRNA extracted according to the method as described above from a cell or a tissue of an appropriate organism.

This invention is also directed to a vector containing the novel gene of the present invention or a fragment thereof, and more preferably, to a vector which contains the cDNA or the genomic DNA of the present invention. Such vector may contain the above-mentioned DNA, and optionally, an enhancer sequence, a promoter sequence, a ribosome binding sequence, a nucleotide sequence used for the purpose of amplifying the number of copies, a nucleotide sequence coding the signal peptide, a nucleotide sequence coding other polypeptide, a polyadenylated sequence, a splicing sequence, a replication origin, nucleotide sequence of the gene acting as selective marker, and the like.

The vector can be produced by incorporating the gene of the present invention as described above in an arbitrary vector by a known method (for example, see Sambrook J. et al., Molecular Cloning, a Laboratory Manual 2nd ed., Cold Spring Harbor Laboratory, New York, 1989). The vector used for the gene incorporation may be appropriately selected from plasmid vectors, cosmid vectors, phage vectors, virus vectors, and the like. When the gene incorporated is a genomic DNA, the vector used should be a vector such as YAC vector, BAC vector, or P1 phage enabling incorporation of an insert with the size of at least several tens to several hundreds kb. When the DNA incorporated is a cDNA, the vector used may be selected from, pUC118, pBR322, pSV2-dhfr, pBluescript II, PHIL-S1, λZap II, λgt10, pAc700, YRP17, pEF-BOS, pEFN-II, and the like.

Of the vector of the present invention, those containing a replication origin, a selective marker, and a promoter in addition to the cDNA of the present invention are those which may be used for the expression of the novel polypeptide of the present invention. The replication origin used may be ColE1, R factor, F factor, or the like when the vector is the one for E. coli, SV40 or the replication origin from adenovirus when the vector is the one for an animal cell, and the replication origin from ARS1 when the vector is the one for yeast. The promoter used may be trp, lac, tac promoter, or the like in the case of E. coli, and in the case of an animal cell, the promoter may be a promotor sequence that is naturally found in the gene of human or other animals such as the promotor from SV40, cytomegalovirus, or adenovirus, or promoter region of elongation factor 1α. The promoter used may be α promoter or the like in the case of yeast, and AOX1 promoter may be used for Pichia pastoris yeast. When such vector is used for the transformation of a eukaryotic cell, an RNA splice-site, a polyadenylation signal, and the like may be added as desired to the sequence as described above.

This invention is also directed to a transformant obtained by conducting the transformation using the vector of the present invention. Such transformant may be obtained by transforming an appropriate host cell with the vector as described above according to a known method (see "Handbook of Genetic Engineering", Special Issue of Experimental Medicine, March 20, 1991, Youdo-sha). The host cell used may be adequately selected from Escherichia coli, Bacillus subtilis, and other prokaryotic cells, and a yeast, an insect cell, an animal cell, and other eukaryotic cells. Preferred examples of the transformant of the present invention are those which are obtained by using E. coli, yeast, or CHO cell for the host and which can express the novel polypeptide of the present invention or a fragment thereof.

This invention is also directed to an antibody which recognizes the novel polypeptide of the present invention or a fragment thereof. Included within the scope of such an antibody are an antibody which specifically recognizes the novel polypeptide of the present invention or a fragment thereof, and also, an antibody which crossreacts with other polypeptides.

The antibody of the present invention is preferably an antibody produced by immunizing an animal through administration of a polypeptide including at least the amino acid sequence shown in Table 1 or 2 or a part thereof which had been optionally bonded with an appropriate carrier such as KLH (keyhole limpet hemocyanine) as the antigen according to a known method (see for example, "Experimental Procedures in Immunology", edited by Japan Immunology Society, issued by Japan Immunology Society). The antigen administered may also be the one expressed as a fusion protein with GST. Both monoclonal and polyclonal antibodies are included within the scope of the antibody of the present invention, and such antibody may belong to any class or subclass.

More preferably, the antibody of the present invention is an antibody which recognizes a polypeptide fragment including the amino acid sequence shown in Table 1 or 2 or a part thereof.

Next, a typical process for producing the antibody of the present invention is briefly described.

First, a polypeptide which is the amino acid sequence shown in Table 1 or 2 or a part thereof and which comprises at least eight amino acid residues is genetically engineered or chemically synthesized, and such polypeptide is bonded to a carrier such as KLH (keyhole limpet hemocyanine) for use as an antigen after purification. The antigen is administered to an animal optionally with an adjuvant such as Freund's complete adjuvant (FCA) or Freund's incomplete adjuvant (FIA), and the animal is boosted at an interval of 2 to 4 weeks. After the booster, blood is collected to thereby obtain the antiserum. The animal immunized is an animal capable of producing the desired antibody, and such animal may be selected from rat, mouse, rabbit, sheep, horse, chicken, goat, pig, cow, and the like. A polyclonal antibody may be produced by purifying the resulting antiserum. The purification may be accomplished by combining known methods such as salting out, ion-exchange chromatography, and affinity chromatography.

The monoclonal antibody may be obtained as described below. An antibody-producing cell such as spleen cell or lymphocyte is collected from the immunized animal, and the collected cell is fused with a myeloma cell or the like by a known method utilizing polyethylene glycol, Sendai virus, electric pulse or the like to thereby produce a hybridoma. A clone producing the antibody which binds to the novel polypeptide of the present invention is selected, and the selected clone is cultivated. The monoclonal antibody is produced from the supernatant of the clone by an adequate combination of known methods such as salting out, ion-exchange chromatography, and affinity chromatography.

The antibody of the present invention may be a neutralizing antibody which inhibits binding of the polypeptide of the present invention to the DNA.

Fab, F(ab'), F(ab')₂, and Fv are also included within the scope of the antibody of the present invention as long as they recognize and bind to the polypeptide of the present invention or a part thereof. Also included within the scope of the antibody of the present invention is a single chain Fv obtained by constructing a gene which codes for a single chain Fv prepared by linking the Fv of H-chain and L-chain and expressing such gene in an adequate host cell. Also included are chimeric antibody, human antibody, and humanized antibody as long as they recognize the polypeptide of the present invention or a part thereof.

The antibody of the present invention can be labeled with a fluorescent substance, an enzyme, a luminescent substance, or a radioisotope for use in detecting the novel polypeptide of the present invention in a cell or nucleus.

Next, the novel method for diagnosing cancer and the novel method for estimating the cancer risk are described.

The novel method for diagnosing cancer of the present invention is a method wherein cell transformation or the possibility of such cell transformation is diagnosed by using the mutation of the novel gene of the present invention for the index. In the present invention, DNA is extracted from the cell or the tissue to be tested, and the DNA is confirmed whether the gene is in its normal state. Alternatively, mRNA may be extracted from the cell or the tissue to be tested, and the mRNA may be confirmed whether the gene has been normally expressed. The "mutation" used herein means replacement, deletion, addition, or insertion of at least one nucleotide. The cell can be evaluated as cancerous or susceptible to cell transformation when it has been found that such replacement, deletion, addition, or insertion of at least one nucleotide has occurred in the novel gene of the present invention or in the chromosomal region including such gene in the test cell, and as a consequence, mutation has been occurred in the amino acid sequence coded, or protein of normal length has failed to be synthesized, or the protein has not been expressed at all expressed. The diagnosing method of the present invention is not limited for its procedure as long as cell transformation or possibility of such cell transformation is diagnosed by using the occurrence of the mutation for the index.

Next, the novel method for estimating the cancer risk of the present invention is described. In this method, risk of cancer development is estimated as an individual's constitution by using occurrence of mutation or polymorphism in the novel gene of the present invention for the index. In the present invention, DNA is extracted from leukocyte in the blood collected from the subject to thereby confirm whether the novel gene of the present invention has undergone any mutation or polymorphism. The "mutation or polymorphism" used herein means replacement, deletion, addition, or insertion of at least one nucleotide. The individual is evaluated to have a constitution with high susceptibility to cancer development (to be highly susceptible to cancer development) when replacement, deletion, addition, or insertion of at least one nucleotide has occurred in the novel gene of the present invention or in the chromosomal region including such gene in the test cell, and as a consequence, the novel polypeptide of the present invention is not at all expressed, or mutation or polymorphism has occurred in the amino acid sequence coded, or protein of normal length is not synthesized. The diagnosis of the present invention is not limited for its procedure as long as the mutation or polymorphism in the novel gene of the present invention is detected and an individual is diagnosed for his/her susceptibility to cancer development by using the occurrence of such mutation or polymorphism for the index.

Next, a typical procedure used in the diagnosing and estimating method of the present invention is described. The procedure employed is substantially the same in the diagnosis and the estimation despite difference in the cells evaluated, and accordingly, the procedure is described in the following for both the diagnosing and estimating methods.

The preferable example of the diagnosing and estimating method of the present invention comprises at least the steps (a), (b), (c), and (d):
(a) extracting DNA from the subject cell or tissue;
(b) producing a PCR primer comprising at least 15 nucleotides which includes the sequence of an arbitrary part of the novel cDNA or genomic DNA of the present invention;
(c) conducting PCR for the DNA extracted in (a) using the primer of (b); and
(d) analyzing the result of the PCR in (c) to determine the occurrence of the mutation or polymorphism in the novel gene of the present invention in the cell of (a).

Alternatively, the preferable example of the diagnosing method of the present invention comprises at least the steps (e), (f), (g) and (h):
(e) extracting mRNA from a cell or a tissue;
(f) producing a PCR primer comprising at least 15 nucleotides which includes the sequence of a part of the novel cDNA or genomic DNA of the present invention;
(g) conducting RT-PCR for the DNA in (e) using the primer of (f); and
(h) analyzing the result of RT-PCR in (g) to determine the occurrence of the mutation or polymorphism in the novel gene of the present invention in the cell of (e).

The steps (a) and (e) in the example as described above may be accomplished by a known method. The primer used in the steps (b) and (f) may be synthesized by a known method. If necessary, the primer used may be labeled with a radioisotope such as ³²P, biotin, horseradish peroxidase, or other known label. When the subject of the diagnosis is human, an oligonucleotide containing a part of the nucleotide sequence shown in Table 4 or the sequence of an antisense strand thereof may be synthesized. Although the number of the nucleotides constituting the primer is not limited as long as the PCR can be accomplished, a primer including 15 or more nucleotides is used in the above-described preferable example in view of the specificity. It is more preferable to use a plurality of primers corresponding to the sequence in the exon 4. The PCR and the RT-PCR of steps (c) and (g) may be carried out by a known method. The analysis conducted in steps (d) and (h) include evaluation of presence/absence of the amplified fragment and length of the amplified fragment, and analysis of the nucleotide sequence of the amplified fragment. To be more specific, occurrence of the mutation or the polymorphism may be determined according to a known method by conducting polyacrylamide gel electrophoresis, agarose gel electrophoresis, pulsed-field gel electrophoresis electrophoresis, or the like depending on the length of the amplified DNA fragment, dying the gel with ethidium bromide, and determining the occurrence of the mutation or the polymorphism from the presence/absence and length of the amplified fragment. Alternatively, sequence of the amplified fragment may be determined by a sequencer. An efficient diagnosis, however, may be accomplished by PCR-SSCP (single strand conformation polymorphism) which is a known method. To be more specific, PCR is conducted by using the primer labeled with ³²P, and the amplified DNA fragment is denatured into single strand DNA by heating and separated by polyacrylamide gel electrophoresis. Position of the bands is detected by autoradiography, and occurrence of replacement, deletion, addition, or insertion at one or more nucleotide in the gene of interest in the test cell can then be detected by comparing the detected band position with the position of the band in the control which has been confirmed for the absence of mutation.

This invention also provides a composition adapted for use in gene therapy produced by using the gene of the present invention.

The composition of the present invention has a characteristic feature that it contains the gene of the present invention, and the composition of the present invention is preferably the gene of the present invention inserted in an adequate vector which enables expression of the gene in the cell. The composition of the present invention is most preferably the one containing the nucleotide sequence which is any one of nucleotide Nos. 11 to 2692 and 149 to 2692 in Table 4, and SEQ ID NO: 11, 13, and 15 in the Sequence Listing.

When γ ray-induced murine lymphoma was analyzed by focusing on the relation between the p53 tumor suppressor gene and the novel tumor suppressor gene of the present invention, it was found that mutation of the novel tumor suppressor gene was reduced in the lymphoma wherein p53 gene was inactive while the mutation was increased in the tumor wherein p53 gene was active. This in turn indicates that there is some overlapping in the function of the novel tumor suppressor gene and the p53, and hence, that the novel tumor suppressor gene may have a function like p53. In other words, the novel tumor suppressor gene may be related to the reverse transformation of the cancer cell or the induction of the cell to death. Accordingly, the cancer cell which has undergone the mutation and wherein the gene has become inactive can be treated by supplementing the particular gene or its translation product, namely, the novel polypeptide of the present invention. In particular, the gene therapy utilizing such gene is believed to be effective if the cancer cell which failed to respond to the gene therapy using the p53. When gene therapy is conducted by using the novel gene, the novel gene of the present invention may be, for example, administered to the patient as a medical composition after incorporating the novel gene in an adequate virus vector such as adenovirus vector or retrovirus vector in the downstream of the expression promoter. If desired, the medical composition may also contain pharmacologically acceptable excipients and additives. The administration method is not particularly limited, and the composition may be directly administered to the tumor cell or the tissue after excision of the tumor.

Next, the present invention is described in further detail by referring to Examples wherein best modes for carrying out the invention are described. These Examples by no means limit the scope of the present invention.

### EXAMPLES

The abbreviations used in the following description are based on the abbreviations commonly used in the art. The procedures in the following Examples were carried out mainly by referring to Sambrook J. et al. ed., Molecular Cloning, a Laboratory Manual 2nd ed., Cold Spring Harbor Laboratory, 1989; and Harlow and Lane, Antibody, a Laboratory Manual, Cold Spring Harbor Laboratory.

### Example 1: Genetic analysis of murine lymphoma

### (1) Production of γ ray-induced lymphoma

An MSM mouse (p53(+/-)MSM mouse) with one p53 gene allele missing was mated with a BALB/c mouse to produce an F1 mouse. When the thus produced F1 mouse reached 4 week old, the mouse was subjected to a fractionated irradiation with γ ray of 2.5 Gy once a week for four times to thereby induce thymic lymphoma. After the irradiation of the γ ray, the mouse (about 15 to 45 week old) showing the symptom of labored respiration was sacrificed, and chest was opened to recover the enlarged thymus tissue. After confirming the occurrence of the lymphoma with naked eye, the tissue was separated with simultaneous washing using physiological saline and pipetting. The thus obtained tissue was used in the following experiments. It is to be noted that morphological change was confirmed by using a part of the tissue.

In the meanwhile, the control normal tissue was prepared by removing brain from the mouse and separating the tissue with simultaneous washing using physiological saline and pipetting.

### (2) Analysis of the gene

The thymic lymphoma produced in Example 1 was homogenized in a homogenizer, and DNA was extracted in a microtube by phenol method according to a known method. The control DNA was also extracted from the brain tissue.

The DNA extract was analyzed for LOH (allelic loss) at 62 loci on all chromosomes. For this purpose, 62 microsatellite markers were prepared according to a known method (Dietrich, WF et al., Nature 380, 149-152, 1996). Using these primers, LOH analysis was conducted by PCR comprising 30 to 35 cycles each comprising heating to 94°C for 1 minute, 55°C for 1 minute, and 72°C for 1 minute. The amplification product obtained by PCR was electrophoresed on 4% Nusieve-agarose gel or 8% polyacrylamide gel, and regions showing LOH at a high frequency were detected in chromosome 11, chromosome 12, and chromosome 16. In chromosome 12, the region showing LOH at a high frequency was the region of about 2.9 cM from D12Mit53 to D12Mit279.

It is to be noted that, unless otherwise noted, PCR was conducted in the following Examples under the conditions as described above.

### (3) Analysis of chromosome 12

PCR was conducted on YAC library (Research Genetics) by using D12Mit53, D12Mit233, D12Mit279, and D12Mit80 as the primers, and as a result, 11 positive clones were obtained. The sequence of a part of the sequence in chromosome 12 included in the thus obtained positive clones was determined by Vectorette PCR (Riley, J et al., Nucl. Acid. Res., 18, 2887-2890, 1990) and sequencing on a sequencer. Sequence tag sites (STSs) markers, namely, Y11(II) and YE6(II) were then prepared on the bases of this sequence. Using these markers, PCR was conducted on the DNA extracted from the γ ray-induced lymphoma produced by the procedure of Example 1. It was then found that the frequency of allelic loss was high between Y11(II) to D12Mit279 (This region is hereinafter referred to as Tlsr4 region).

Analysis of the YAC library revealed that the YAC clone fully including the Tlsr4 region was not yet obtained. In order to make a physical map fully covering the Tlsr4 region, PCR using Y11(II), YE6(II), YC3(II), D12Mit233, and D12Mit279 for the primers was conducted for BAC library (Research Genetics).

There were then obtained 15 positive clones.

The clones obtained were ligated as described below. DNA was extracted from the positive clones and digested with NotI, and the digestion products were subjected to pulsed field gel electrophoresis (PFGE) according to a known method, and there was then confirmed a fragment of about 15 kb to 160 kb. When the NotI digestion products were analyzed by southern hybridization using a fragment of about 20 kb obtained by digesting B2N clone with NotI for the probe, clones B3N, B8L, B50N, and B50F were found to include the 20 kb fragment. In the meanwhile, primers were prepared by determining the terminal sequence of the clones, and PCR was conducted to other clones to thereby examine the overlapping between the clones. It was then confirmed that 5 clones including the B2N covered the region in which allelic loss had commonly occurred in lymphoma (referred to as the Tlsr4 region), and that the region covered was about 340 kb in total.

A part of the nucleotide sequence of these clones were determined, and further STSs markers were prepared for use as polymorphism markers (B19E(I), MCA1, B2N(I), B3N(II), B3K(I)). Using these markers, LOH was analyzed by conducting PCR for the DNA extract from the γ ray-induced lymphoma produced by the procedure of Example 1. It was then found that the Tlsr4 region is located in the region of about 35 kb between B2N(I) and B3N(II).

One NotI restriction enzyme site is located in this region. The NotI site is located in CpG sequence, and presence of the gene near the CpG sequence was indicated.

### Example 2: Identification and analysis of tumor suppressor gene

### (1) Identification of tumor suppressor gene

DNA was extracted by a known method from B8L clone including the Tlsr4, and the DNA was fragmented with a sonicator. The resulting fragments (those having the size of about 2 kb) were incorporated in pUC118 vector. The pUC clones were randomly selected, and sequenced. About 800 nucleotides are sequenced at one sequencing, and such process was repeated about 500 times. The resulting sequences were subjected homology search one by one, and homology was confirmed for 16 ESTs sequences (ID Nos: AI060775, AI152076, AA739203, AA200063, AA738980, AA939664, AA512234, WI30385, AA072284, AA118486, AA896738, AA930957, AA466966, AI174109, and AA474814) corresponding to 4 sequences. Analysis was conducted by using a program which estimates the exon (GRAIL), and as many as 50 regions had the possibility of being an exon.

The following procedure was conducted by focusing on one of the regions and assuming that the region focused was the exon. The region focused was located near the Tlsr4 region (at a location about 40 kb from the NotI site), and had a long open reading frame (ORF), and also, 6 zinc finger motifs were coded in the amino acid sequence of this region. The inventors of the present invention have formerly identified Ikaros gene as a lymphoma suppressor gene on chromosome 11, and this Ikaros was also a gene having 6 zinc finger motifs despite difference in the arrangement of the zinc finger motifs. As a consequence, it was assumed that the sequence coding for the zinc finger motifs which was confirmed in this procedure was a part of the candidate gene.

In order to determine the full length cDNA sequence including this region, the following two procedures were conducted. First, examination was conducted to determine which one of the ESTs sequences and the candidate sequences for the exon were actually linked in the cDNA. For such examination, PCR primers were prepared on the bases of the EST sequences and exon sequence, and RT-PCR was conducted for the mRNA extracted from the thymus of the normal mouse. While most primers failed to produce PCR products, PCR products were detected in some of the primer combinations (for example, FW49-2 and RA2). Two new exons were revealed by ligating these sequences. PCR was also conducted for ligation with a part of the potent candidate gene whose nucleotide sequence had been determined. The resulting sequence was the one coding about 850 amino acids. The remaining exons were determined by 5' RACE (Frohman et al., 1988, Pro. Natl. Acad. Sci., vol.85, 8998-9002) using 5' RACE system (GibcoBRL, Version 2). The primers used were RW-49, R'Y802, and R'RW49-1. As a consequence, an ORF coding 884 amino acids in total and extending over 4 exons was confirmed. In other words, nucleotide sequence of the full length cDNA was determined (Table 5, mouse Sequence Listing and Table 3). The exon having the zinc finger motifs corresponded to the fourth exon. It was also found that the genomic structure of this gene was such that exon 1 was located at about 1.5 kb from the NotI site, and the overall size was about 45 kb. It was then revealed that exons 1 and 2 were located in the Tlsr4 region.

### (2) Analysis of the protein coded by the gene

On the bases of the thus obtained nucleotide sequence of the gene, structure of the protein coded by such gene was analyzed. The amino acid sequence deduced from the nucleotide sequence is indicated in Table 1, and it was the second ATG codon that matched the Kozak's rule, and the possibility was conceived that, in some cells, the polypeptide coded by such gene was expressed as the polypeptide having an amino acid sequence in the downstream of 47th methionine (M) in Table 1. It was also found that the novel polypeptide had a zinc finger of C₂H₂ type (class I type) (Table 1, FIG. 1). This protein was designated Rit1. Zinc finger is a DNA-binding motif which is found in many transcription regulators, and it was then conceived that Rit1 may be involved in the organism with transcription regulation. As shown in Table 1, Rit1 has three zinc finger clusters. The cluster on the side of the N terminal comprises a single zinc finger, and the second zinc finger cluster includes two zinc fingers, and the third cluster includes three zinc fingers (referred to as the 2-3 type). A proline-rich region is located between the first zinc finger cluster and the second zinc finger cluster, and a region rich in acidic amino acids is located between the second zinc finger cluster and the third zinc finger cluster. It was estimated that these regions contribute for the promotion of transcription. A structurally unique character of the Rit1 protein is that it has a structure different from most of the known transcription factors having the C₂H₂ zinc finger wherein the zinc fingers are concentrated in one location (Sp/XKLF type). There are also proteins which are known to have the zinc fingers located at two or more separate locations (for example, Ikaros), and in all of such proteins, the zinc finger motifs are arranged such that three zinc finger motifs are located in the zinc finger cluster on the N terminal side, and two zinc finger motifs are located in the zinc finger cluster on the C terminal side (referred to as the 3-2 type), and such structure is different from that of the Rit1. Accordingly, the zinc finger structure of 2-3 type confirmed in Rit1 is believed to be an utterly new structure.

### Example 3: Confirmation of mutation in tumor cell

### (1) Homozygous deletion assay

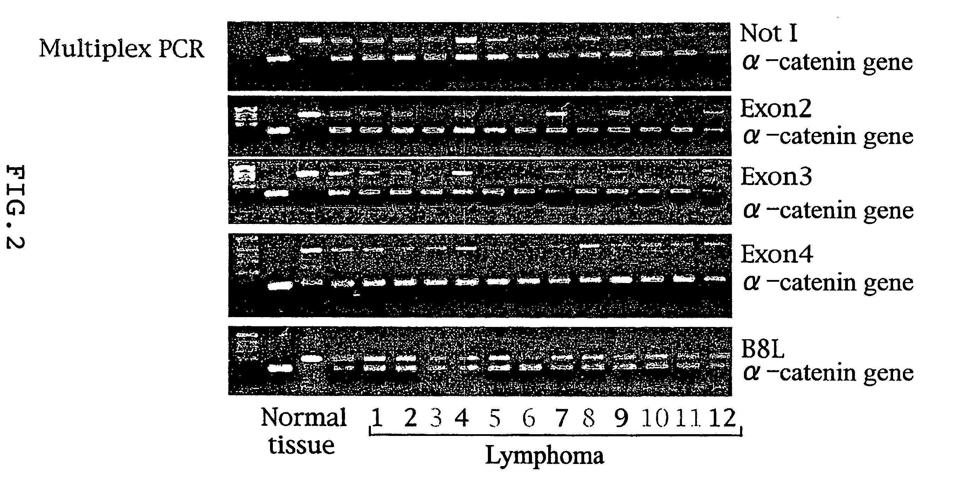

Further analysis was conducted for the 146 cases of mouse thymic lymphoma wherein allelic loss was found in the analysis of Example 2 using B19E(I), MCA1, B2N(I), B3N(II), and B3K(I) for the markers. First, four sets of primers (NotIF and NotIR2, FW49 and RW49-2, FM717 and RM717, and FZ-Zinc and RZ-Zinc) were prepared on the bases of the sequences of the region near the NotI site in the upstream of exon 1, exon 2, exon 3, and exon 4. Multiplex PCR was conducted for the DNA extracted from the murine thymic lymphoma as described above by using these primer sets and B8L(II) primer. The internal standard gene used was alpha-catenin gene. Multiplex PCR was also conducted in a similar manner for the control, the DNA extracted from the brain tissue. As a consequence, partial loss in the Rit1 gene region was recognized in 14 cases out of 146 thymic lymphoma cases. Of these cases, the results for 6 cases are shown in FIG. 2 (the cell of Nos. 3, 4, 6, 8, 10, and 11 in FIG. 2). In FIG. 2, loss was found in the region extending from exon 2 to exon 3 in five cases (Nos. 3, 5, 8, 10, and 11), and loss was found in the region extending from exon 2 to B8L(II) in No. 6.

Next, mRNA was extracted by a known method from the tumor cells wherein partial loss of both Rit1 gene alleles was found, and expression of the mRNA was examined by RT-PCR. For this purpose, a primer having homology with the sequence on the 3' terminal side of exon 2, and two sets of primers having homology with the sequence on 5' terminal side of exon 4 were prepared (FW49-2 and RW514, FM714 and RM526). The RT-PCR was conducted by 36 cycles of reaction each comprising heating to 96°C for 1 minute, 55°C for 30 seconds, and 72°C for 30 seconds. The resulting products were separated and identified by polyacrylamide gel electrophoresis. The results were such that the normal expression of Rit1 gene was clearly missing in all cases.

### (2) Analysis of the occurrence of mutation

The cases of tumor wherein no deletion/loss of the Rit1 gene was confirmed in (1) were analyzed for the occurrence of mutation in the Rit1 gene. In this analysis, the second and the third zinc finger cluster regions were used as the region of the mutation detection. PCR was conducted by using primers capable of amplifying the lymphoma genomic DNA and the zinc finger cluster region. The nucleotide sequence of the PCR products obtained were determined. As a consequence, point mutation in exon 3 was confirmed in 1 specimen, and point mutation in exon 4 was confirmed in 4 specimens. It was also confirmed that all mutations induced the mutation at the level of amino acid in the protein. The mutation in exon 3 was deletion of 22 nucleotides. One mutation in exon 4 was the replacement of C (cysteine) in the zinc finger motif at 849th from the N terminal with S (serine), and other mutations were replacement of 679th K (lysine) from the N terminal with T (threonine), replacement of 723th R (arginine) with C (cysteine), and deletion of 39 nucleotides. The results of (1) and (2) indicate that the change in Rit1 matches the two-hit model of Knudson (Knudson, AG, Proc. Natl. Acad. Sci. USA, 68, 820-824, 1971), and it was conceived that Rit1 is functioning as a tumor suppressor gene in the organism. It was also decided from the results of the analysis that, in most cases of mouse thymic lymphoma, the Rit1 gene is either not expressed at all or even if expressed, has undergone a mutation.

### (3) Expression of Rit1 gene in various tissues

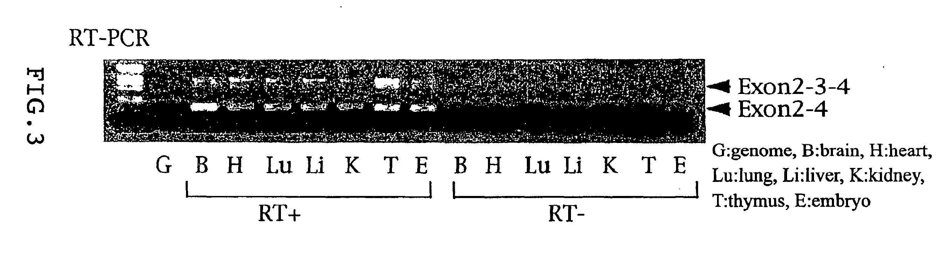

RT-PCR was conducted using the two primers having homology with the sequence on the 3' terminal side of the exon 2 prepared in (1) and having homology with the sequence on the 5' terminal side of the exon 4 (FW49-2 and RA2) for the mRNA extracted from mouse brain, heart, lung, liver, kidney, thymus, and embryo. Expression of the Rit1 gene was confirmed in all of these tissues, indicating the expression of Rit1 gene in a broad range of tissues (FIG. 3). On the other hand, in the Northern blot analysis using the cDNA containing the sequence corresponding to exons 1, 2 and 4 of the Rit1 gene, clear expression was recognized in thymus and brain. These results indicate that Rit1 gene usually functions as a tumor suppressor gene mainly in thymus and other lymphatic cells and brain and other neural cells although Rit1 gene is expressed in many tissues and cells.

Existence of isotypes was indicated by the DNA length of the amplified bands, and further analysis of the sequence confirmed presence of three isotypes, namely, the one having exons 1-2-3-4 (referred to as Rit1α), the 1-2-4 type wherein exon 4 is connected in the downstream of exon 2 (referred to as Rit1β), and the mutant 1-2-3-4 type having exons 1-2-3-4 except that about 12 nucleotides on the 3' terminal side of exon 3 has been spliced out (referred as Rit1α'). Apart from these, there was also confirmed a 1-4 isotype wherein exon 4 is connected in the downstream of exon 1 (referred as Rit1γ). γ type was expressed to a higher degree in lymphoma than in thymus. Since exon 4 was included in all isotypes, it was conceived that exon 4 plays the central role in the tumor-suppressing activity.

### Example 4: Identification and analysis of the gene in human

### (1) Identification of human Rit1 gene

A human EST which has homology with a part of the genomic sequence near the mouse Rit1 gene was found in homology search (ID No. WI30385). It was also found that this human EST is mapped in 14q32 region of human chromosome 14. This region in human is the region corresponding to Rit1 locus of mouse. In view of the situation as described above, primers were prepared on the bases of the sequence of this EST (F-385 and R385), and a commercially available human genomic library (Research Genetics) was screened by PCR. The resulting human BAC clones were analyzed for their nucleotide sequence on opposite ends to thereby produce STSs markers, which were used in isolating a series of adjacent BAC clones.

The BAC clone DNA was amplified by PCR using primers (FW49 and RW49-2) produced on the bases of the sequence of exon 2 of the mouse Rit1 gene, and primers (F-HE505 and R-HE505) produced on the bases of the human EST (ID No. AA333505) showing homology with the Rit1. As a consequence, amplification of the DNA corresponding exons 2 and 4 of the mouse was found in two clones. The DNA was extracted from the two positive clones, and digested with EcoRI, BamHI, and HindIII. The resulting DNA fragments were incorporated in Charomid vector to prepare a library. The library was screened by the two primer sets as described above, and the subclones whose amplification was recognized were determined for their nucleotide sequence. The thus obtained nucleotide sequence was compared with the nucleotide sequence of mouse Rit1 gene, and the nucleotide sequence of the region which was believed to be the exon 2 and the exon 4 in human Rit gene was thereby identified. Primers corresponding to the 5' terminal sequence of the exon 2 and the 3' terminal sequence of the exon 4 (FW823 and RW514) were prepared, and RT-PCR using these primers was conducted using the mRNA extracted from human thymus for the template. By determining the sequence of the resulting cDNA, substantially full sequence of the human Rit1 gene (cDNA) was determined, and comparison with the mouse sequence revealed that this sequence was the region corresponding to exons 2 to 4. Next, the procedure of Example 2(1) was repeated by conducting 5' RACE for the mRNA extracted from human thymus, and cDNA nucleotide sequence including the exon 1 was confirmed. A probe was prepared on the bases of this sequence, and the BAC clones as described above were screened. The nucleotide sequence of the human Rit1 gene (cDNA) including exons 1 to 4 was thus determined (Table 6, human Sequence Listing, Table 4). Analysis of the amino acid sequence coding for the human Rit1 gene as described above revealed that this sequence had the zinc finger structure of 2-3 type as in the case of mouse (Table 2). It was also found in the case of human that it was the second ATG codon that matched the Kozak's rule as in the case of mouse, and the possibility was conceived that, in some cells, the polypeptide coded by such gene was the polypeptide having an amino acid sequence in the downstream of 47th methionine (M) in Table 2.

### Example 5: Construction of expression vector

A primer (F-EX1) was produced on the bases of the sequence of exon 1 with HindIII site added on the 5' terminal, and another primer (R-EX1) was produced on the bases of the sequence of exon 4, and RT-PCR was conducted for the mRNA extracted from mouse thymus. The resulting amplification product was digested with PstI to recover a fragment of about 730 bp, which was incorporated in pUC118. This fragment included the region from the upstream of the initiation codon of the exon 1 to the 5' terminal side of the exon 4. In the meanwhile, the clones containing the gene of interest were digested in BAC clones with restriction enzymes to recover the amplification product. For example, the clones including all of B2N(I), MCA1, and B8L(II) serving as markers were digested with PstI and EcoRI, and the PstI-PstI (about 1 kbp) and the PstI-EcoRI (about 1.5 kb) were recovered as amplification products. The pUC118 as described above was digested with PstI, and these amplification products were sequentially ligated using DNA ligase. With regard to human, primers were also prepared on the bases of the sequence of the exon region of the human Rit1, and RT-PCR was conducted for the mRNA extracted from human thymus. The amplified fragment was ligated and incorporated in the pUC118 as described above to thereby obtain the expression vector.

### Example 6: Production of Rit1 protein-recognizing antibody

### (1) Production of the antigen to be administered

A chimeric polypeptide having a part of the amino acid sequence coded by Rit1 gene on the C terminal side of glutathione-S-transferase (GST) (Smith, D. B. & Johnson, K. S., Gene, vol.67, 31-40, 1988) was prepared by the procedure as described below. First, a primer set was designed such that BamHI restriction sequence is located on the 5' terminal side and EcoRI restriction sequence is located on the side of the 3' terminal side of the DNA fragment coding for the amino acid of nucleotide Nos. 1343 (coding Ser) to 1710 (coding Ala) in mouse Rit1 DNA shown in Table 3. By using these primers, the DNA fragment amplified by RT-PCR in Example 2 was amplified by PCR, and the amplified fragment was recovered and digested with BamHI and EcoRI. In the meanwhile, a plasmid, pGEX2T (Pharmacia) containing the cDNA for GST was digested with BamHI and EcoRI, and developed on 1% agarose gel (low melting point agarose, Takara) for recovery. The DNA fragment as described above was inserted in the digested plasmid. An E. coli strain, DH5a was transformed with this plasmid according to a known method.

The resulting E. coli transformant was cultivated in L-broth supplemented with 10 mg/ml ampicillin and 0.1mM IPTG, and the chimeric polypeptide was produced in a large amount in the E. coli by inducing the expression. E. coli was then suspended in PBS (150mM NaCl, 16mM Na₂HPO₄, 4mM NaH₂PO₄, 0.1mM PMSF) supplemented with 1% Triton, and after the ultrasonication, the supernatant was recovered by centrifugation. The supernatant was then passed through glutathione Sepharose column (Pharmacia) for adsorption of the chimeric peptide, which was eluted with an elusion buffer (50mM Tris-HCl, pH 8.0, 5mM glutathione) for use as an antigen.

### (2) Preparation of antiserum

The antigen for administration produced in (1) (1 mg/0.5 ml PBS) was mixed with an equal amount of RIBI that has been reconstituted with PBS (MPL + TDM + CWS emulsion, Funakoshi), and the mixture was subcutaneously and intracutaneously administered to a rabbit (12 week old, female). After 4 weeks, the rabbit was boosted twice with 500 µg of the antigen at an interval of 4 weeks, and antiserum was obtained by collecting the blood and separating the serum.

### (3) Affinity purification of antiserum

The chimeric polypeptide that had been expressed in E. coli and solubilized in (1), and GST which is not fused with the peptide were bonded to glutathione-agarose beads, respectively, and the beads were washed with 0.2M sodium borate (pH 9.0). Dimethyl pimelimidate (final concentration, 20mM) was added to the beads for irreversible bonding of the antigen to the beads to thereby produce chimeric polypeptide-affinity beads and GST-affinity beads, respectively.

Next, the antiserum which had been diluted ten times with 10mM Tris-HCl (pH 7.5) was mixed first with the GST-affinity beads for removal of the anti-GST antibody by adsorption. The supernatant was then mixed with the chimeric peptide-affinity beads for adsorption of the anti-Rit1 antibody. The beads were then washed with 10mM Tris, pH 7.5, 500mM NaCl, and eluted with 100mM glycine for the recovery of the purified anti-Rit1 antibody.

### (4) Production of monoclonal antibody

50 µg of the antigen produced in (1) was diluted with physiological saline to 0.1 ml, mixed with an equal amount of Freund's complete adjuvant (DIFCO), and administered to abdomen of a Wistar rat (female, 8 weeks old). After two weeks, the same amount of antigen that had been mixed with Freund's incomplete adjuvant (DIFCO) was administered in a similar manner. After boosting for several times, final administration of the antigen was conducted 3 days before the cell fusion. Lymphocyte was aseptically collected from spleen of the rat, and the collected lymphocyte was mixed with myeloma cell SP2/O-Ag14 for cell fusion according to a known method (c.f. Ando, T and Chiba, T, "Introduction to Experimental Procedures of Monoclonal Antibodies" Kodansha Scientific). The supernatant was collected after 6 days. The antigen peptide was insolubilized on a plate for selection of an antibody-producing hybridoma that produces an antibody specifically binding to the insolubilized peptide. The antibody-producing hybridoma was then cloned by limiting dilution analysis (c.f. Ando, T and Chiba, T, "Introduction to Experimental Procedures of Monoclonal Antibodies" Kodansha Scientific), and screening on the peptide-solubilized plate was again conducted to obtain the hybridoma that produces the monoclonal antibody reacting with the peptide.

The monoclonal antibody was produced by precultivating the hybridoma in RPMI-1640 supplemented with 10% bovine fetal serum, and then cultivating in Hybridoma-SFM (GIBCO) to 3 L. The resulting supernatant was passed through a filter paper to remove the cells, and added to a protein A column, Prosep-A (Millipore). The column was washed with 0.1M phosphate buffer (pH 7.5) containing 0.15M NaCl, and eluted with 0.1M glycine-HCl buffer (pH 3.0) for elution of the monoclonal antibody that had been bonded to the column. After bringing the pH of the eluate back to neutral, the eluate was concentrated by Diaflow (Grace Japan) and dialyzed against 0.076M phosphate buffer containing 0.45% NaCl to obtain the desired monoclonal antibody.

Tables 1 to 4 presented below are to show the sequences determined in the Examples.

<Table 1> The amino acid sequence of novel polypeptide (Rit1) from mouse determined in Example 2(1) is shown in Table 1(1) and (2).

<Table 2> The amino acid sequence of novel polypeptide (Rit1) from human determined in Example 4 is shown in Table 1(1) and (2).

<Table 3> The nucleotide sequence of the cDNA of the novel gene (Rit1 gene) from mouse determined in Example 2(1) is shown in Table 3(1), (2), (3), (4), and (5). The parts corresponding to exons 1 to 4 and open reading frame are marked with an arrow at their starting position, respectively. With regard to the coding region, the position of the initiation codon and the position just before the termination codon are respectively indicated with an arrow. The parts coding for the zinc finger motif are indicated with underline. The proline-rich region is indicated with double underline, and the acidic amino acid region is indicated with wavy line.

<Table 4> The nucleotide sequence of the cDNA of the novel gene (Rit1 gene) from human determined in Example 4 is shown in Table 4(1), (2), (3), and (4). The parts corresponding to exons 2 to 4 are marked with an arrow at their starting position, respectively. With regard to the coding region, the position of the initiation codon and the position just before the termination codon are respectively indicated with an arrow. The parts coding for the zinc finger motif are indicated with underline. The proline-rich region is indicated with double underline, and the acidic amino acid region is indicated with wavy line.

<Table 5> Sequence Listing for mouse is shown as Table 5(1), (2), (3), (4), and (5).

<Table 6> Sequence Listing for human is shown as Table 6(1), (2), (3), (4), and (5).

<Table 7> The sequence of mouse Rit1α' is shown. The DNA sequence shown corresponds to the DNA of Sequence Listing, SEQ ID NO: 5, and the amino acid sequence shown corresponds to the polypeptide of Sequence Listing, SEQ ID NO: 6.

<Table 8> The sequence of mouse Rit1β is shown. The DNA sequence shown corresponds to the DNA of Sequence Listing, SEQ ID NO: 7, and the amino acid sequence shown corresponds to the polypeptide of Sequence Listing, SEQ ID NO: 8.

<Table 9> The sequence of mouse Rit1γ is shown. The DNA sequence shown corresponds to the DNA of Sequence Listing, SEQ ID NO: 9, and the amino acid sequence shown corresponds to the polypeptide of Sequence Listing, SEQ ID NO: 10.

<Table 10> The sequence of human Rit1α' is shown. The DNA sequence shown corresponds to the DNA of Sequence Listing, SEQ ID NO: 11, and the amino acid sequence shown corresponds to the polypeptide of Sequence Listing, SEQ ID NO: 12.

<Table 11> The sequence of human Rit1β is shown. The DNA sequence shown corresponds to the DNA of Sequence Listing, SEQ ID NO: 13, and the amino acid sequence shown corresponds to the polypeptide of Sequence Listing, SEQ ID NO: 14.

<Table 12> The sequence of human Rit1γ is shown. The DNA sequence shown corresponds to the DNA of Sequence Listing, SEQ ID NO: 15, and the amino acid sequence shown corresponds to the polypeptide of Sequence Listing, SEQ ID NO: 16.

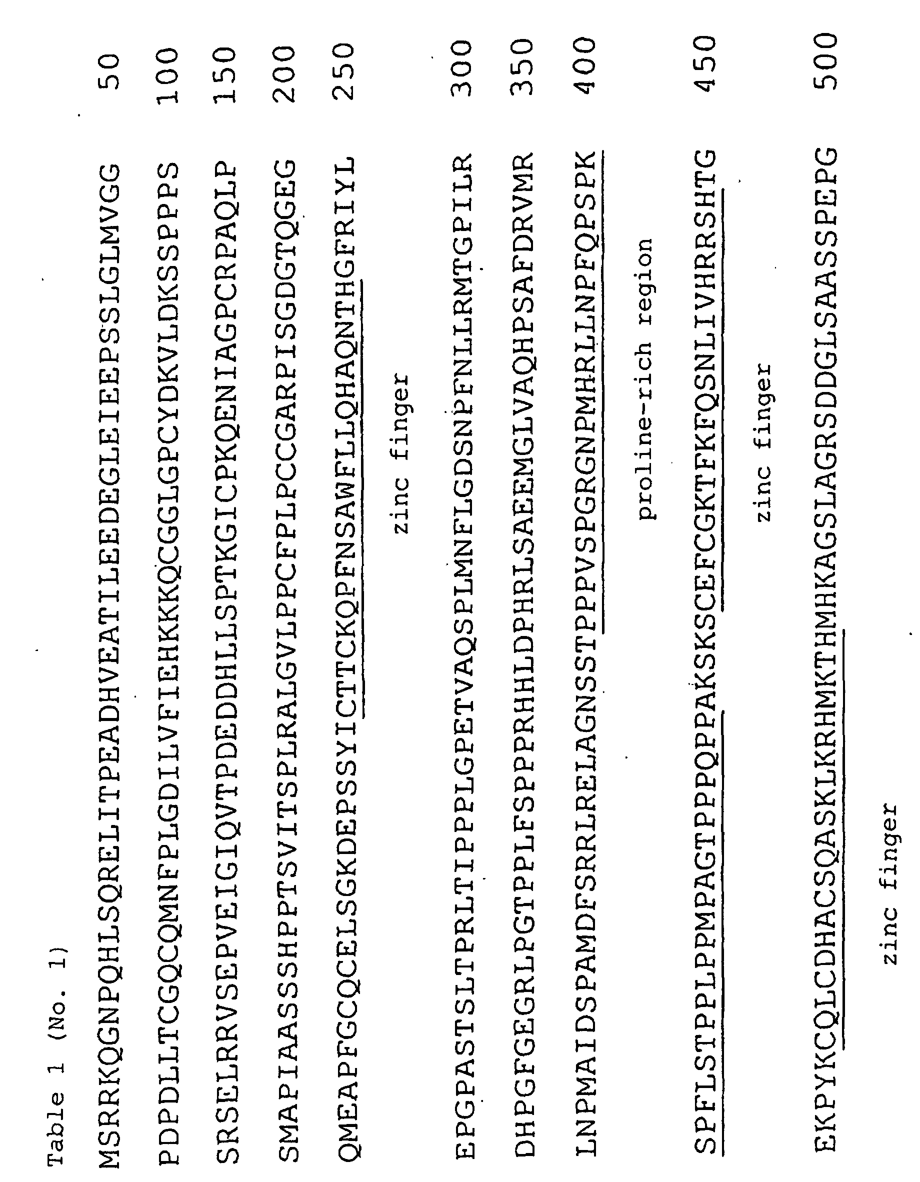

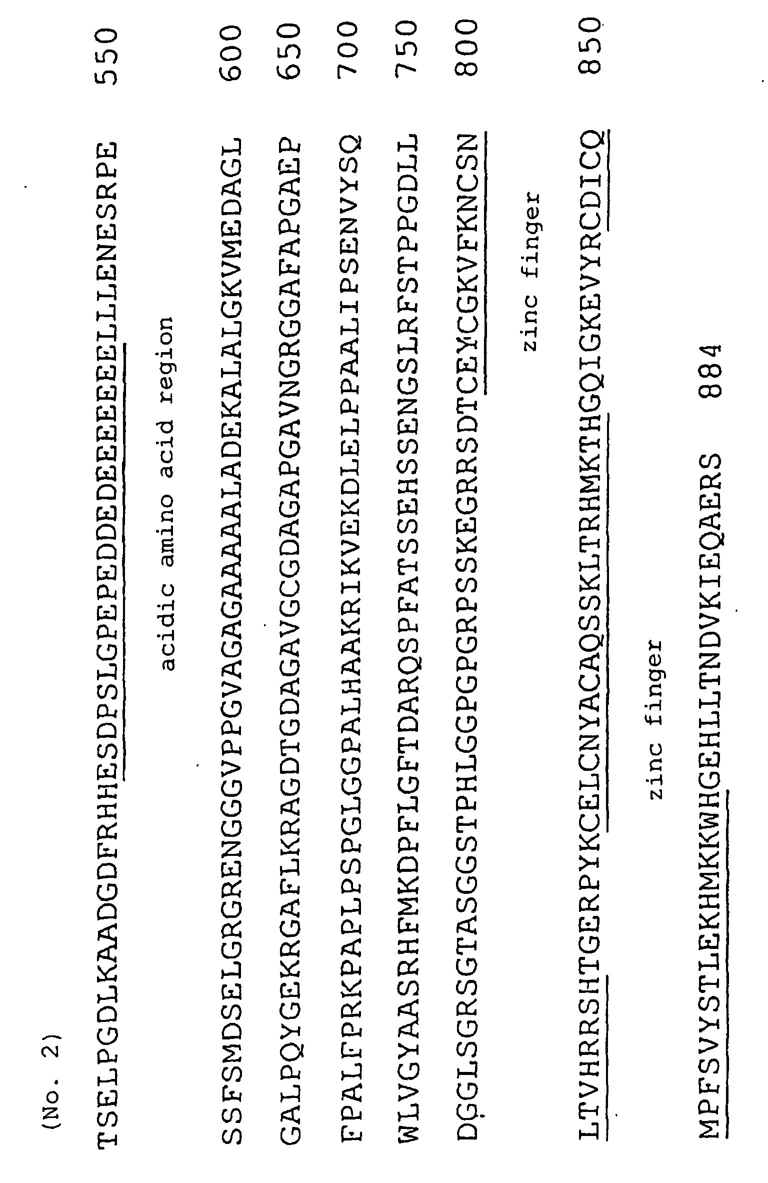

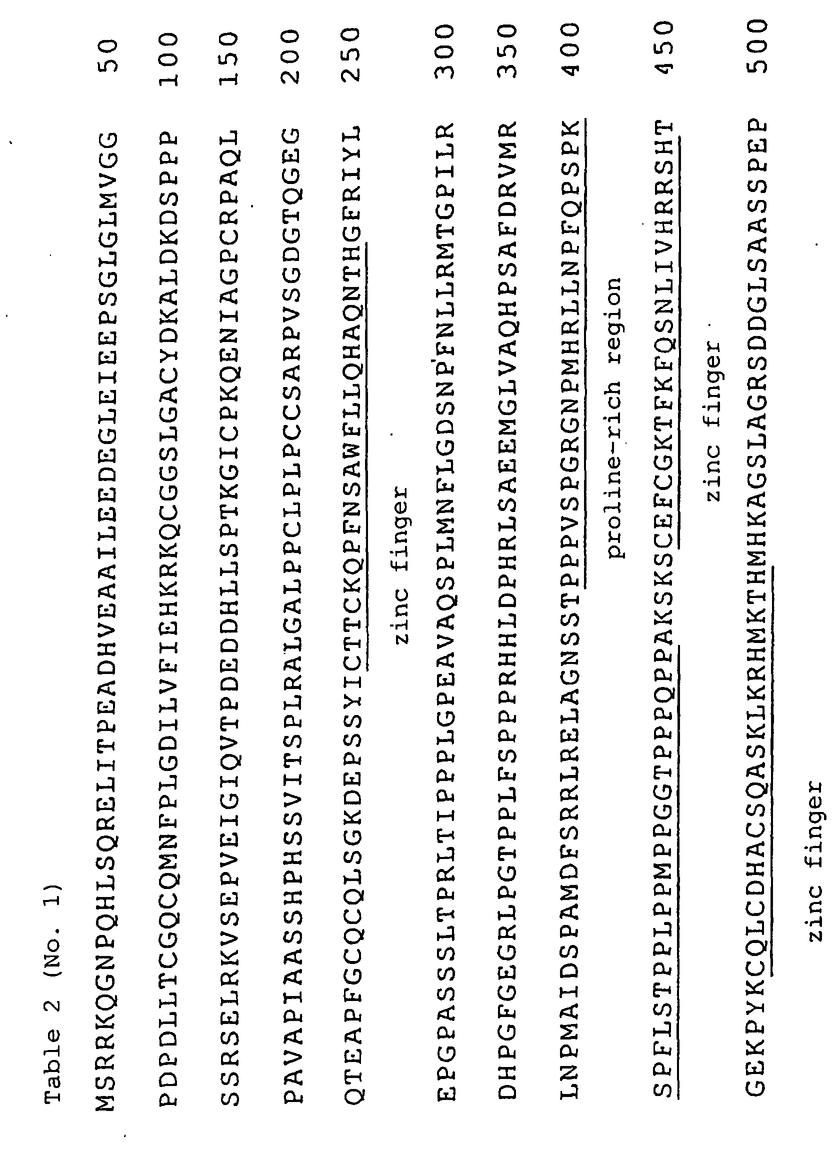

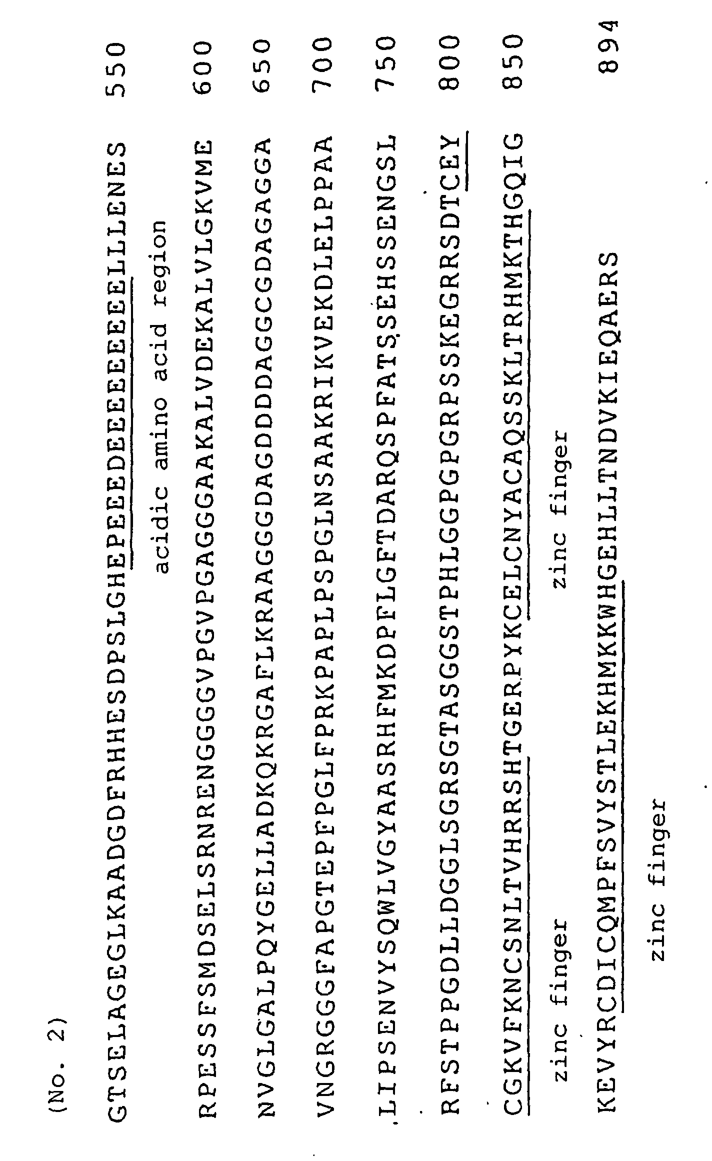

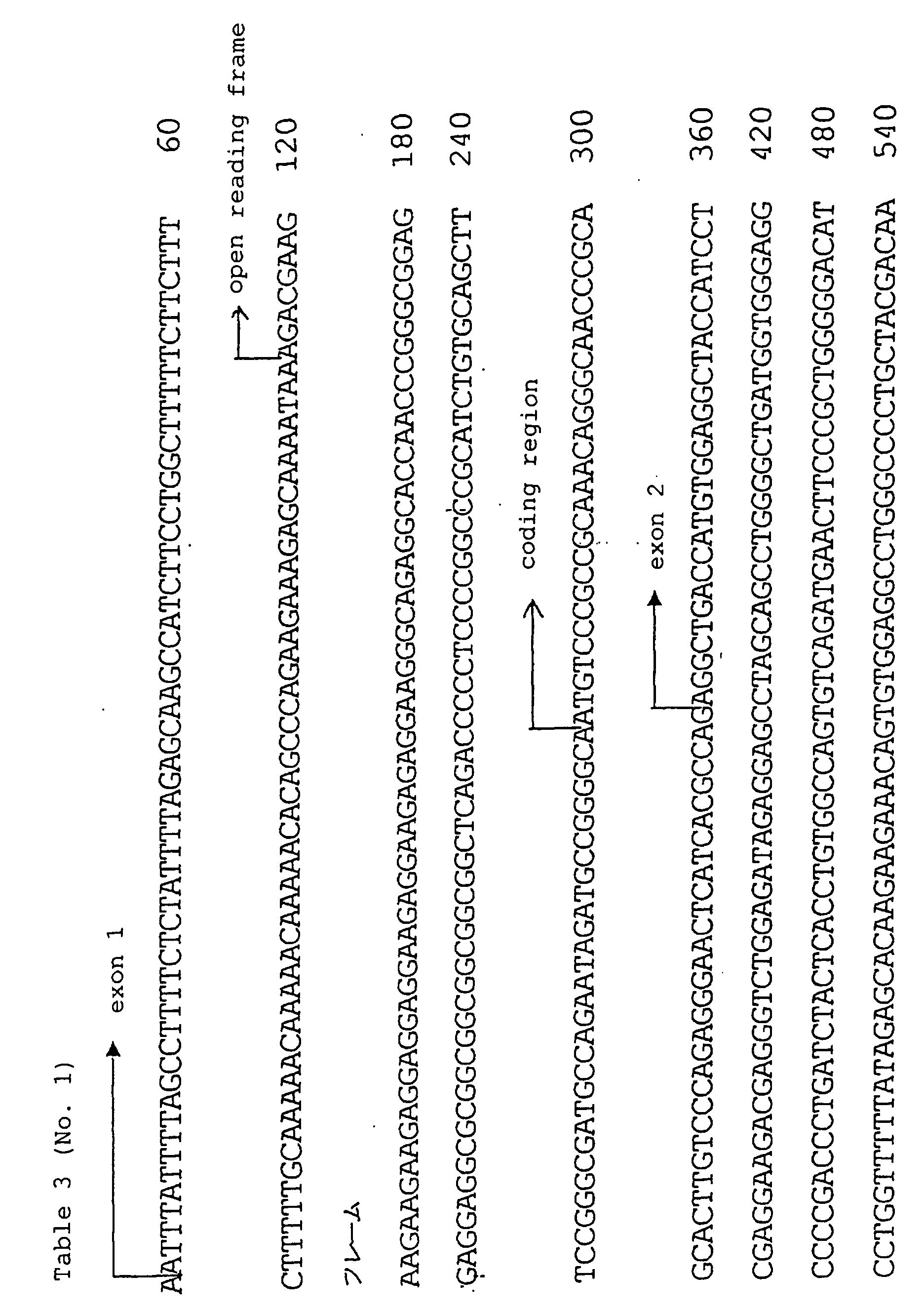

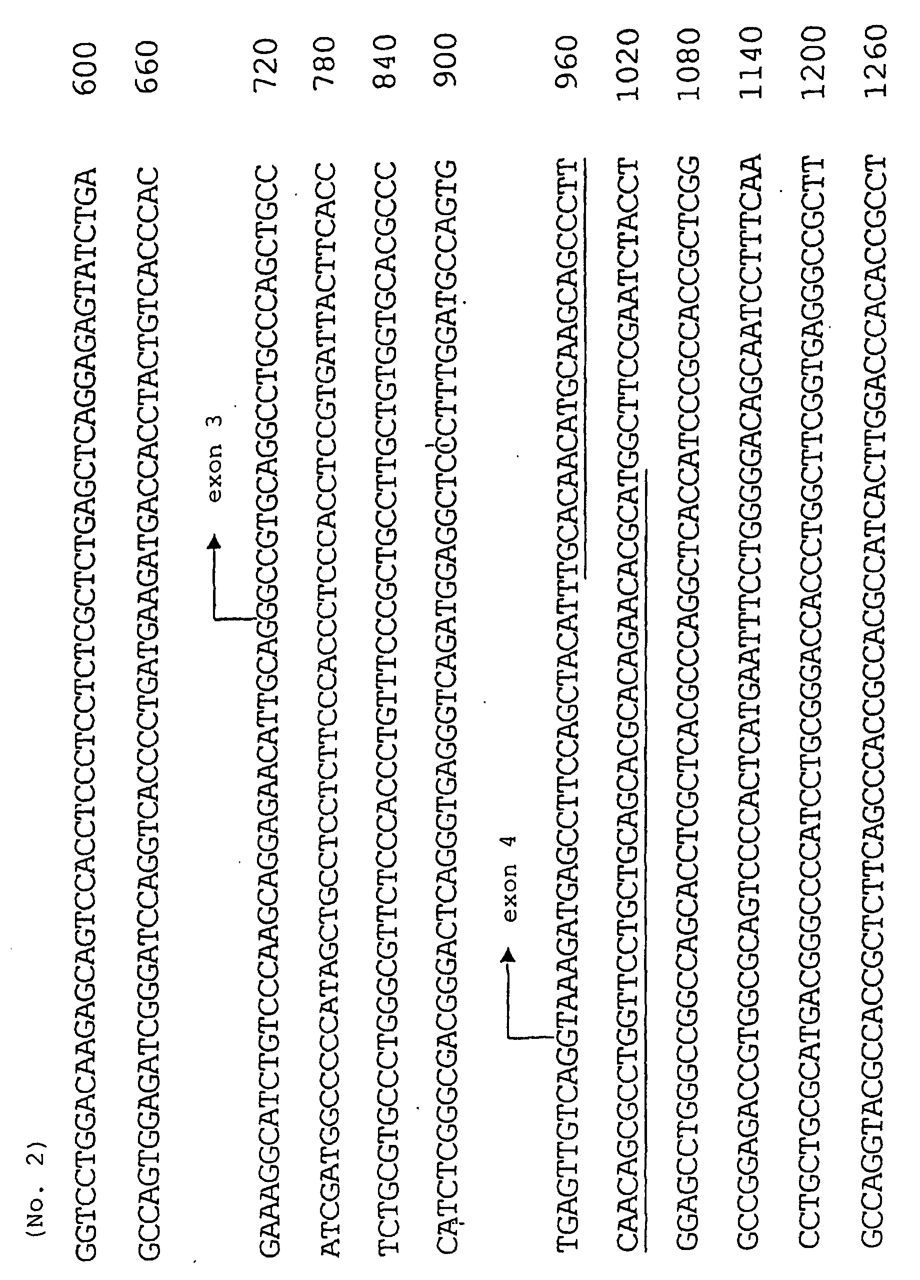

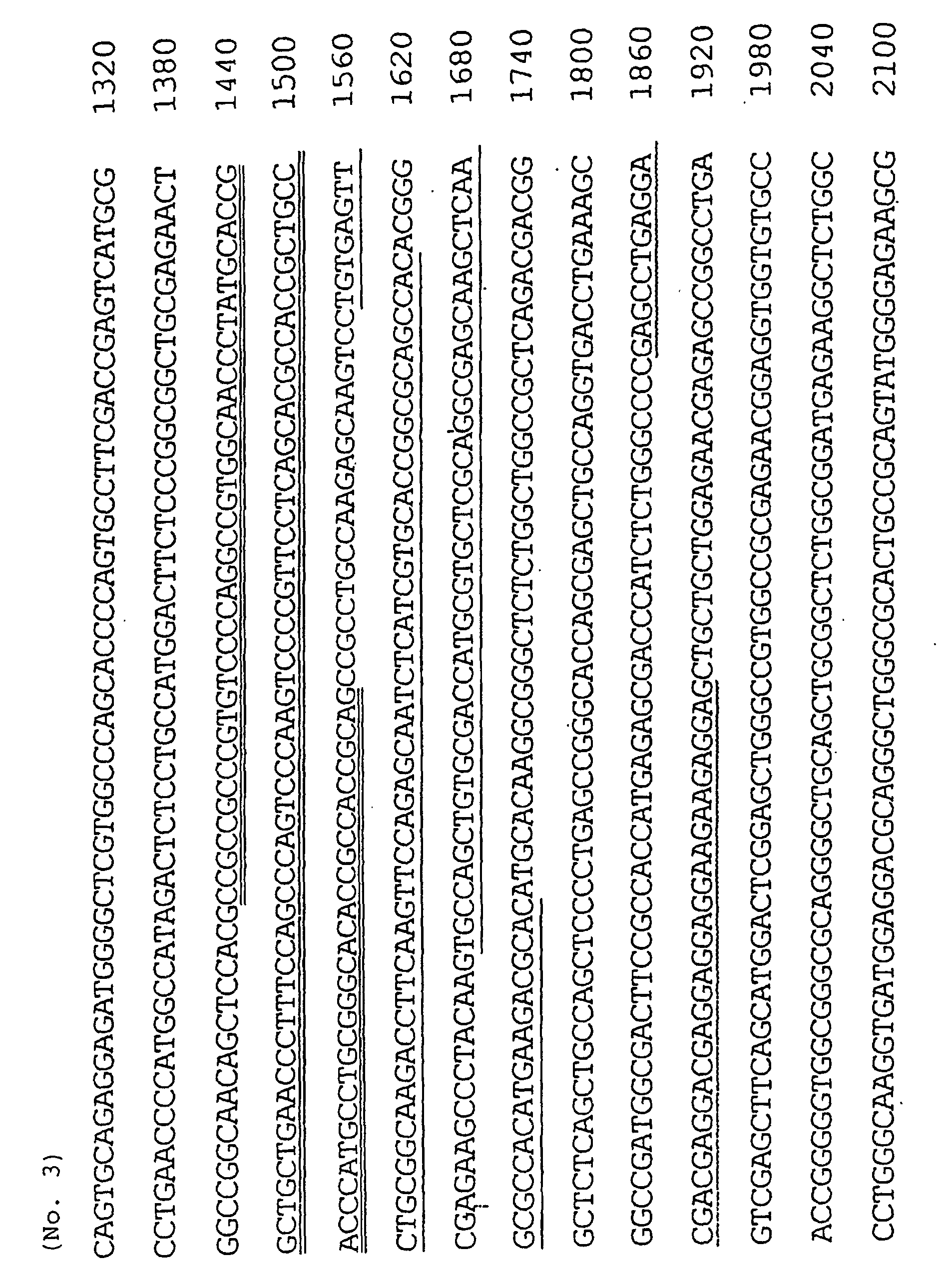

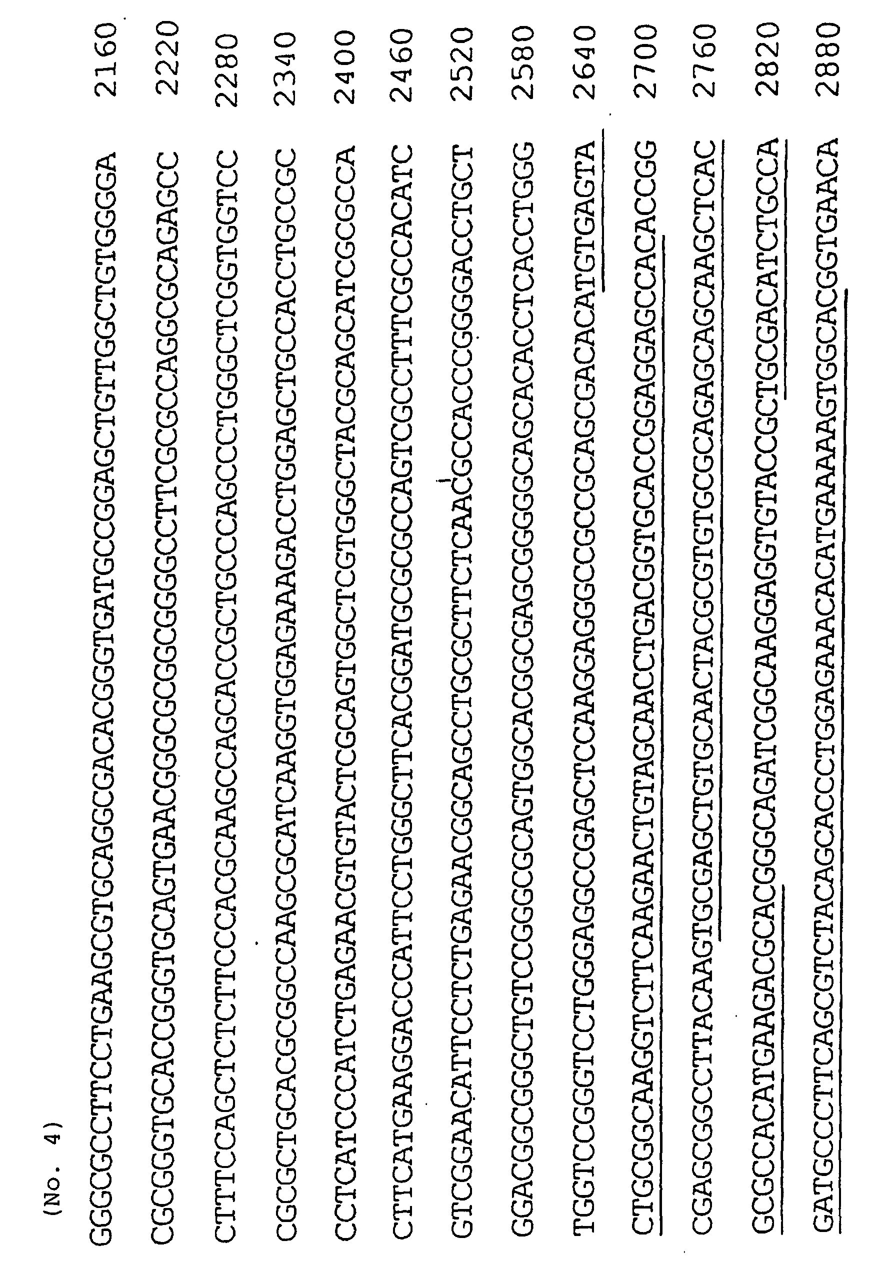

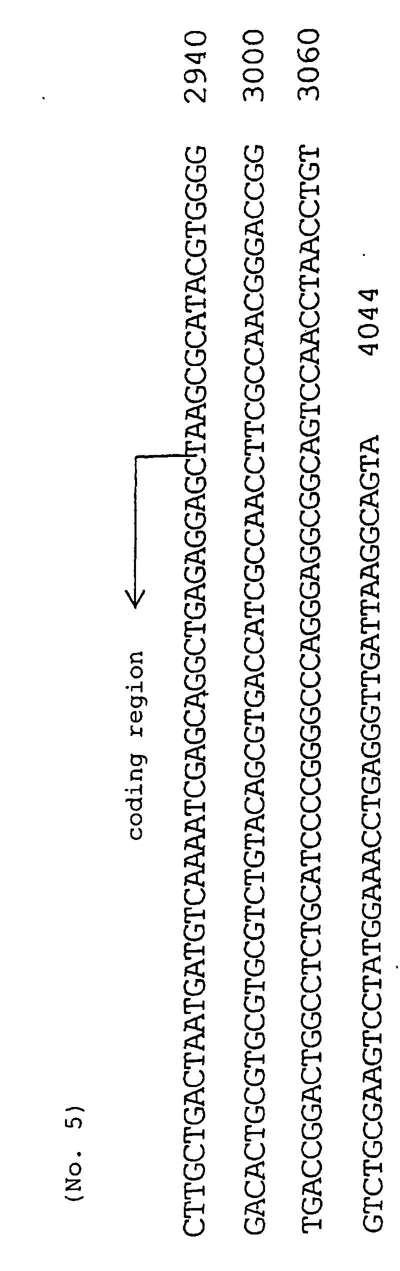

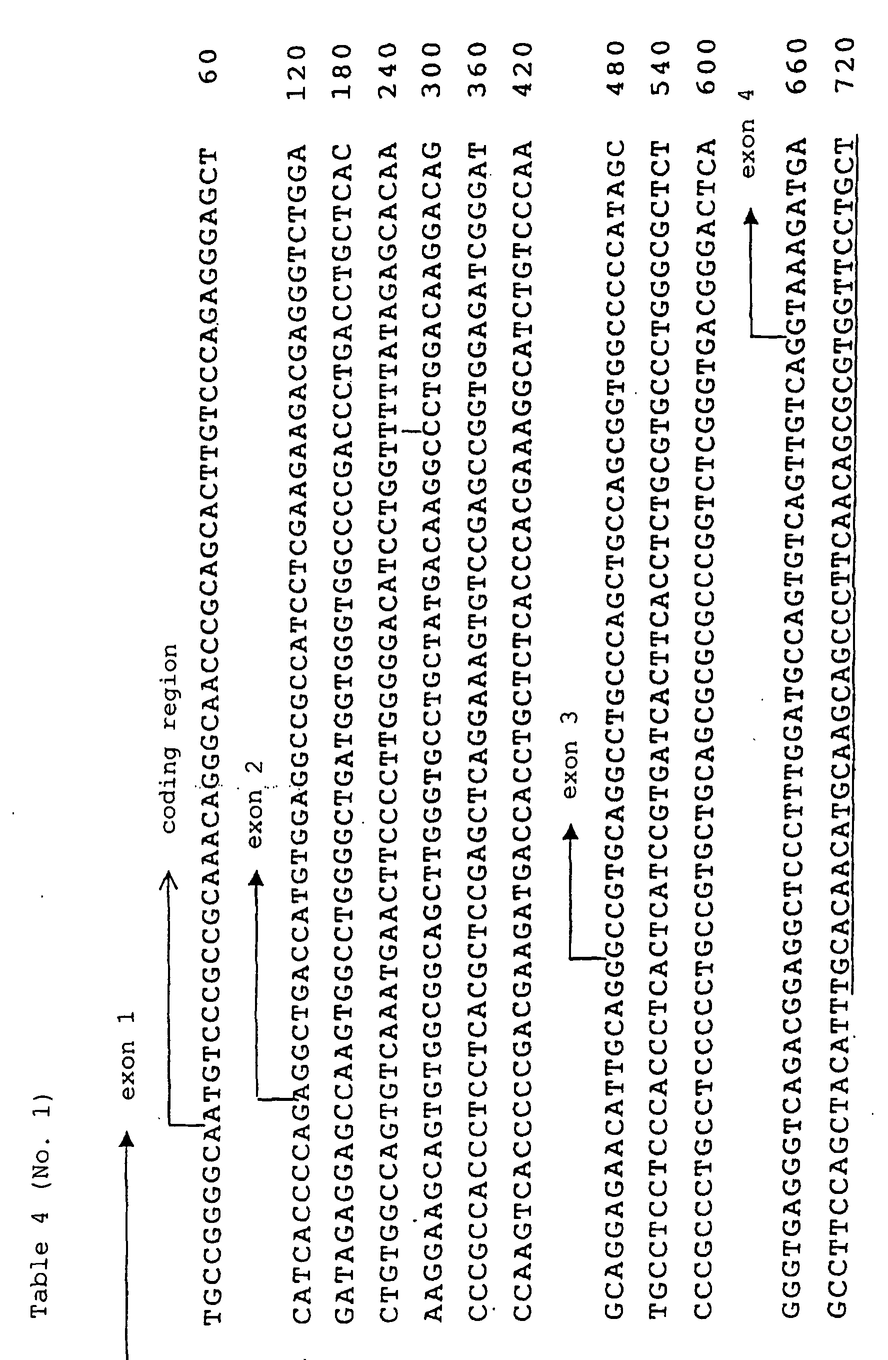

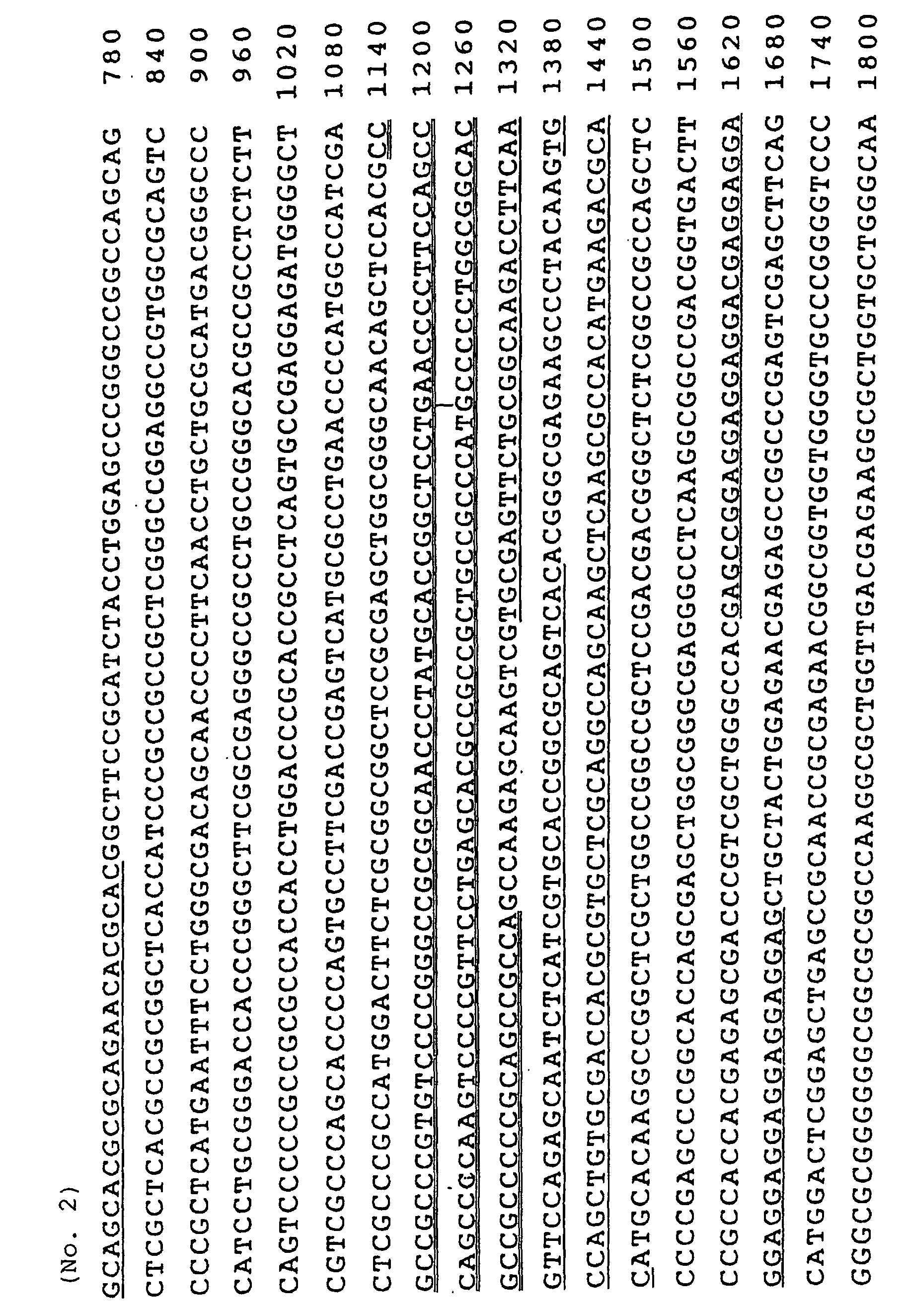

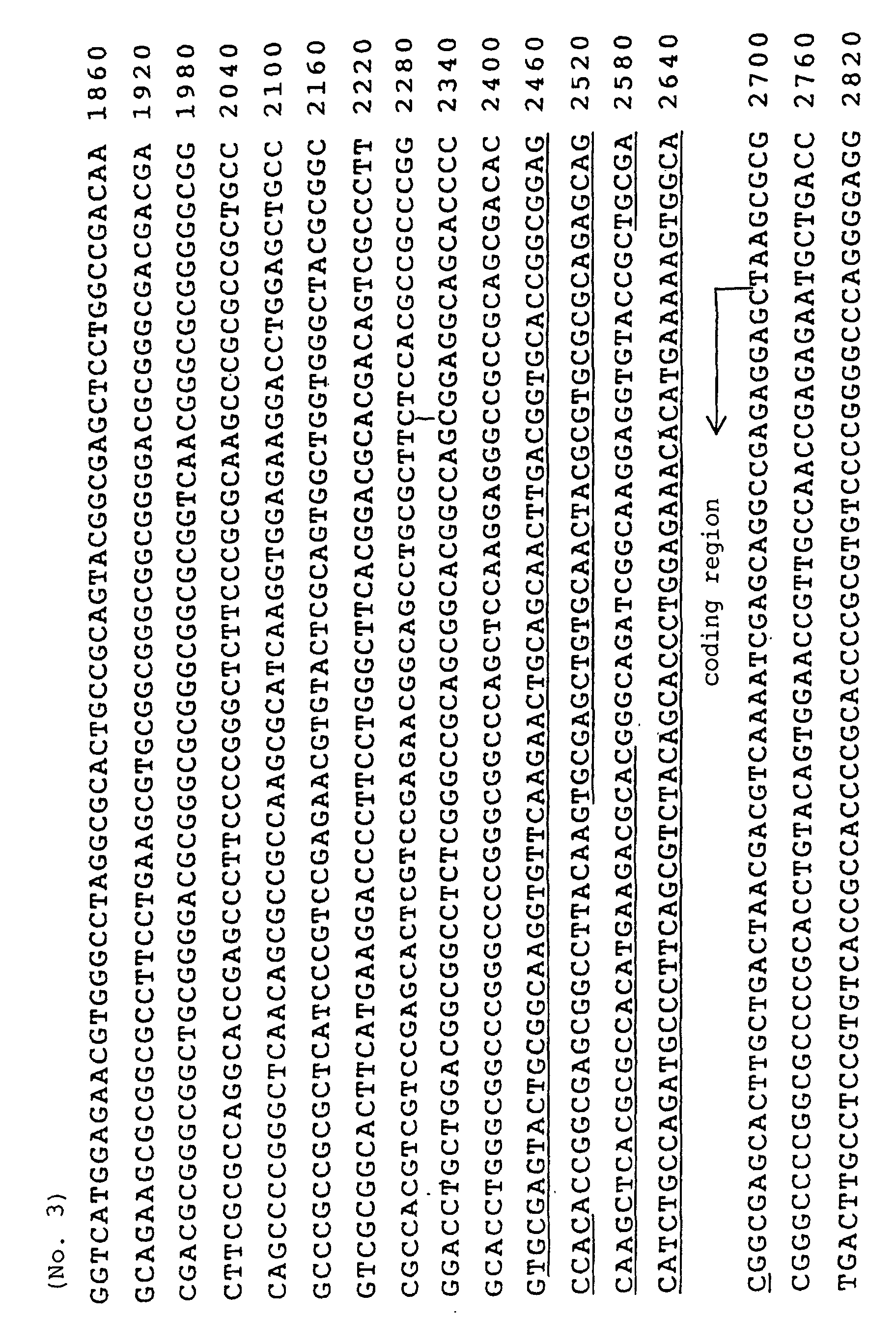

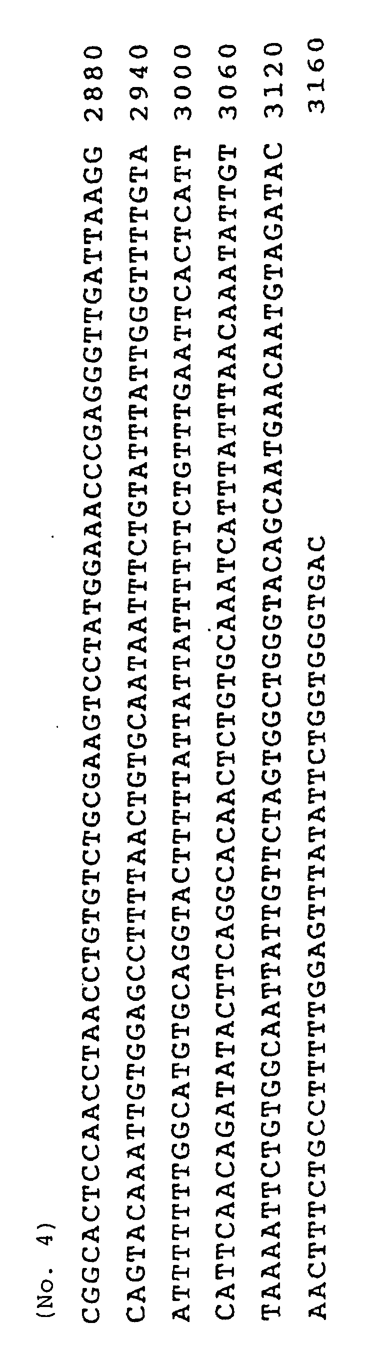

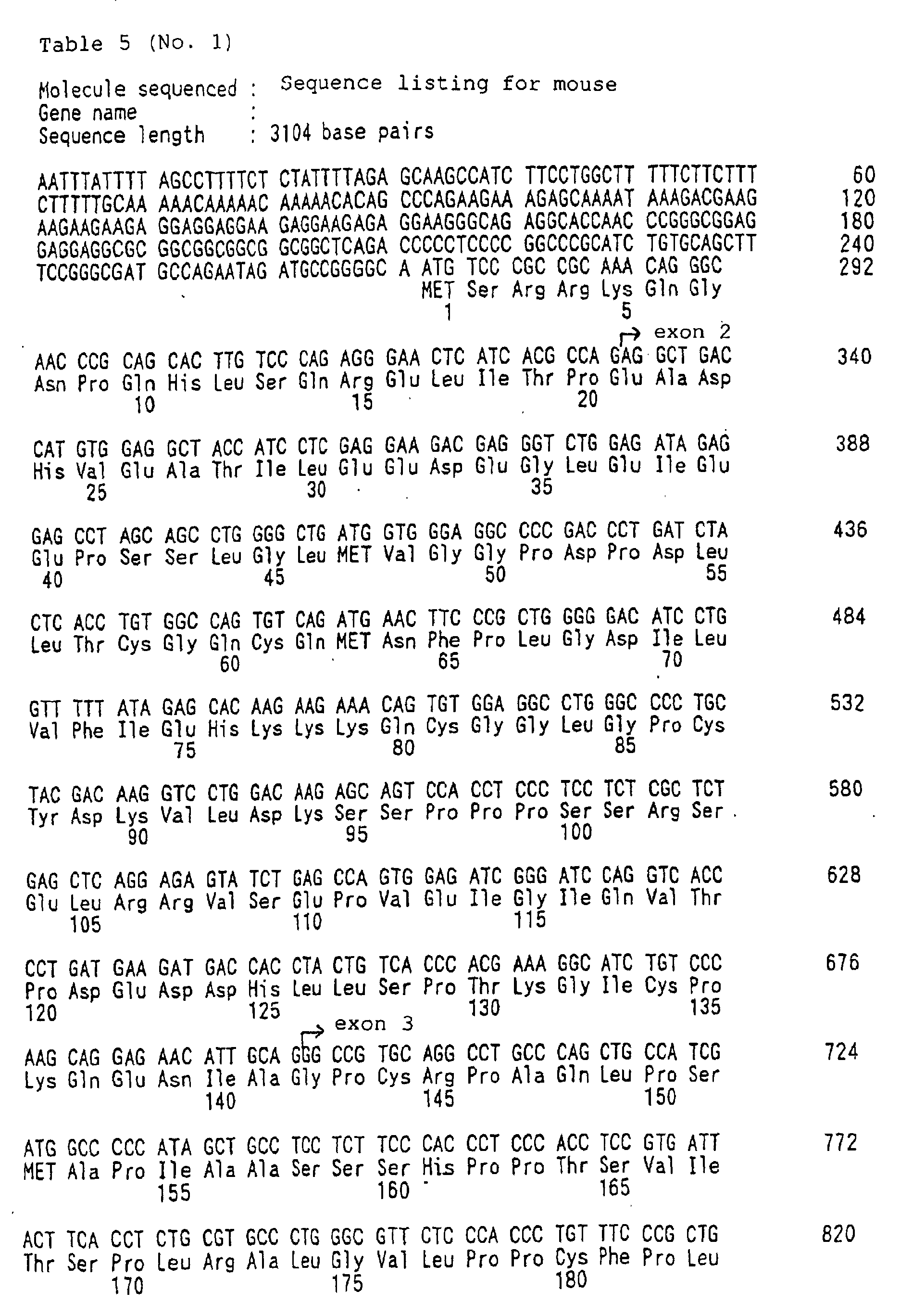

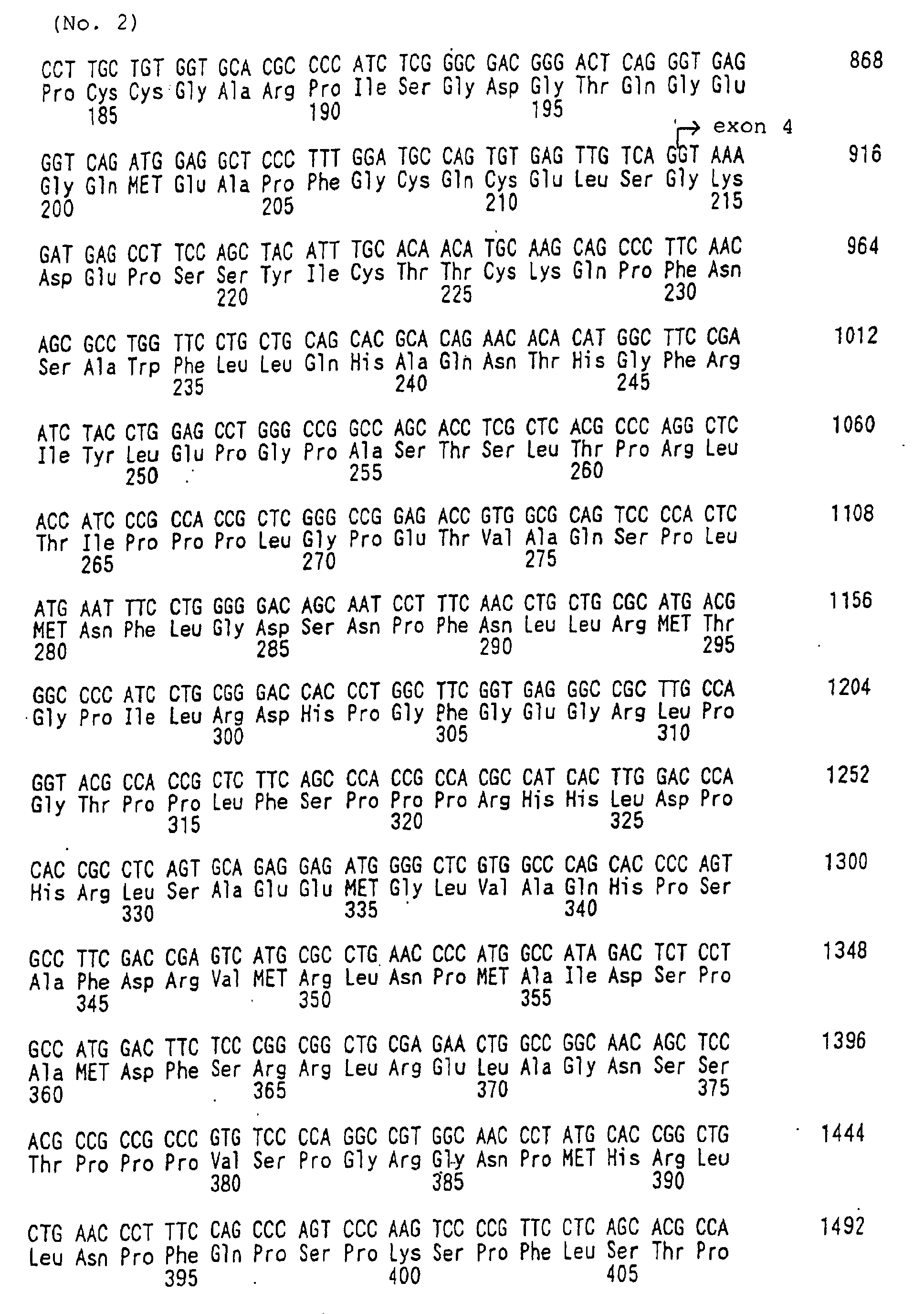

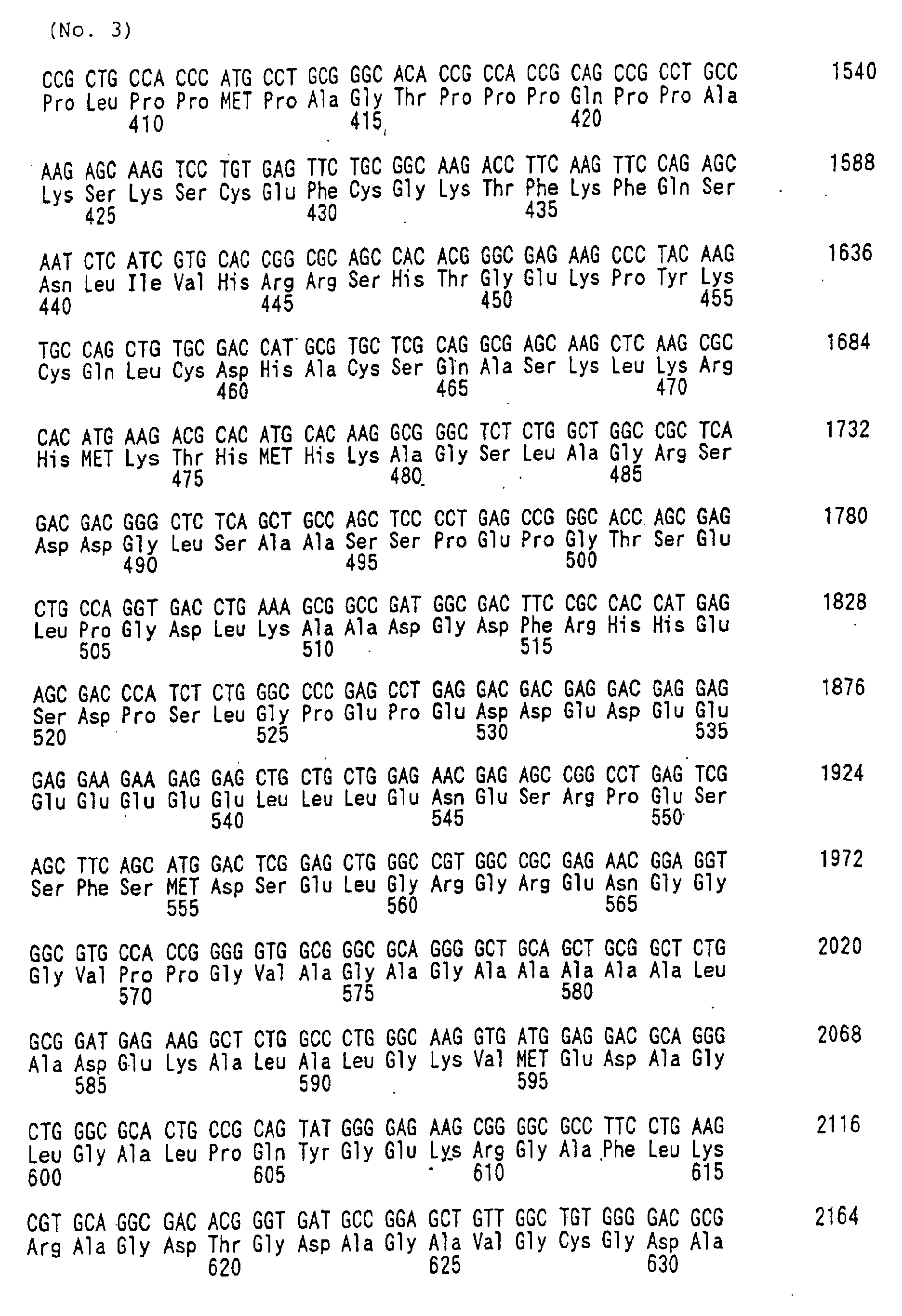

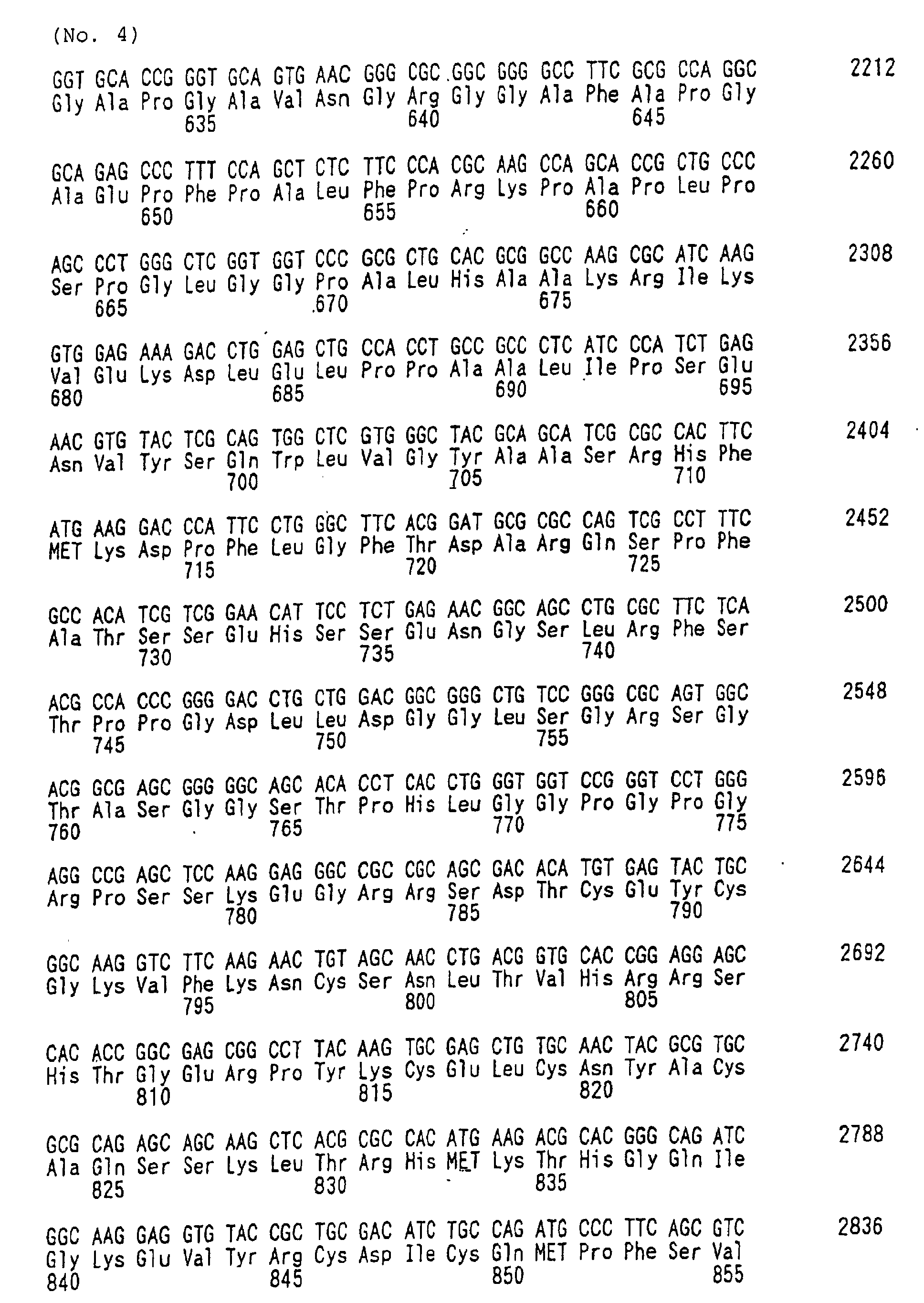

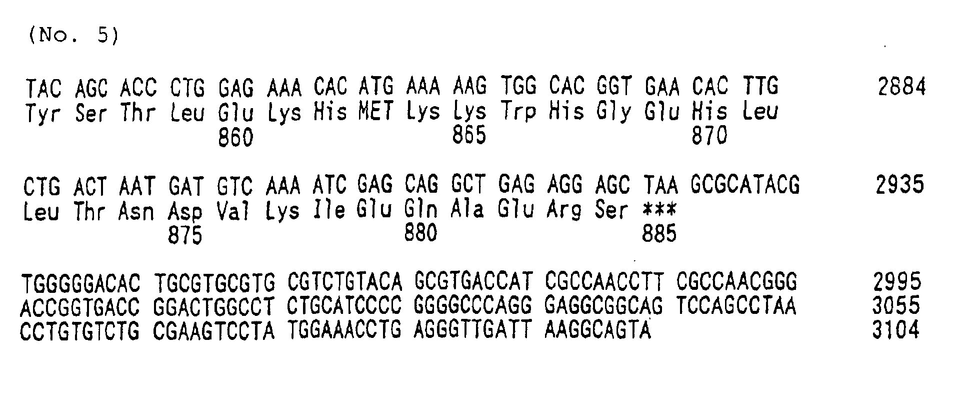

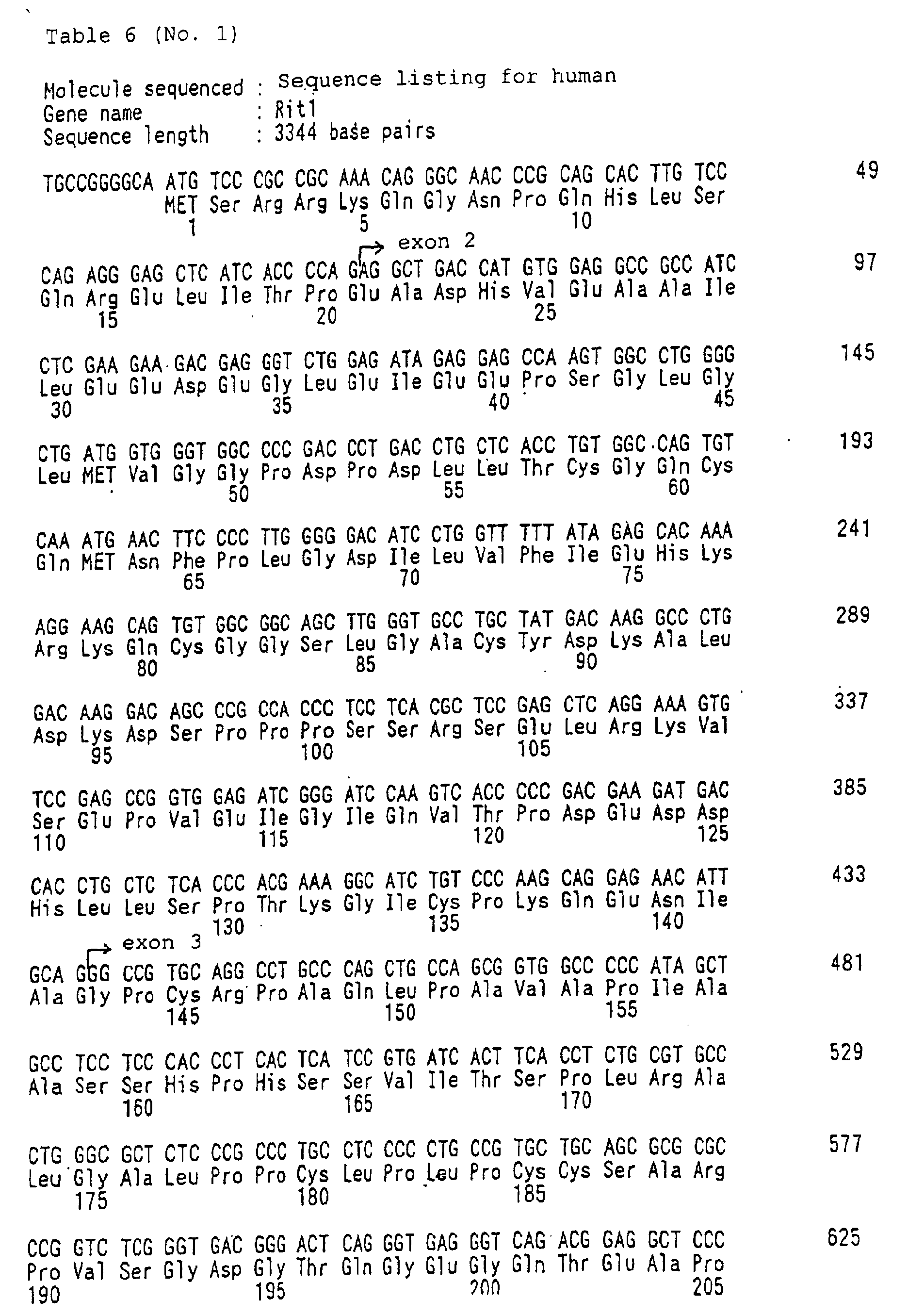

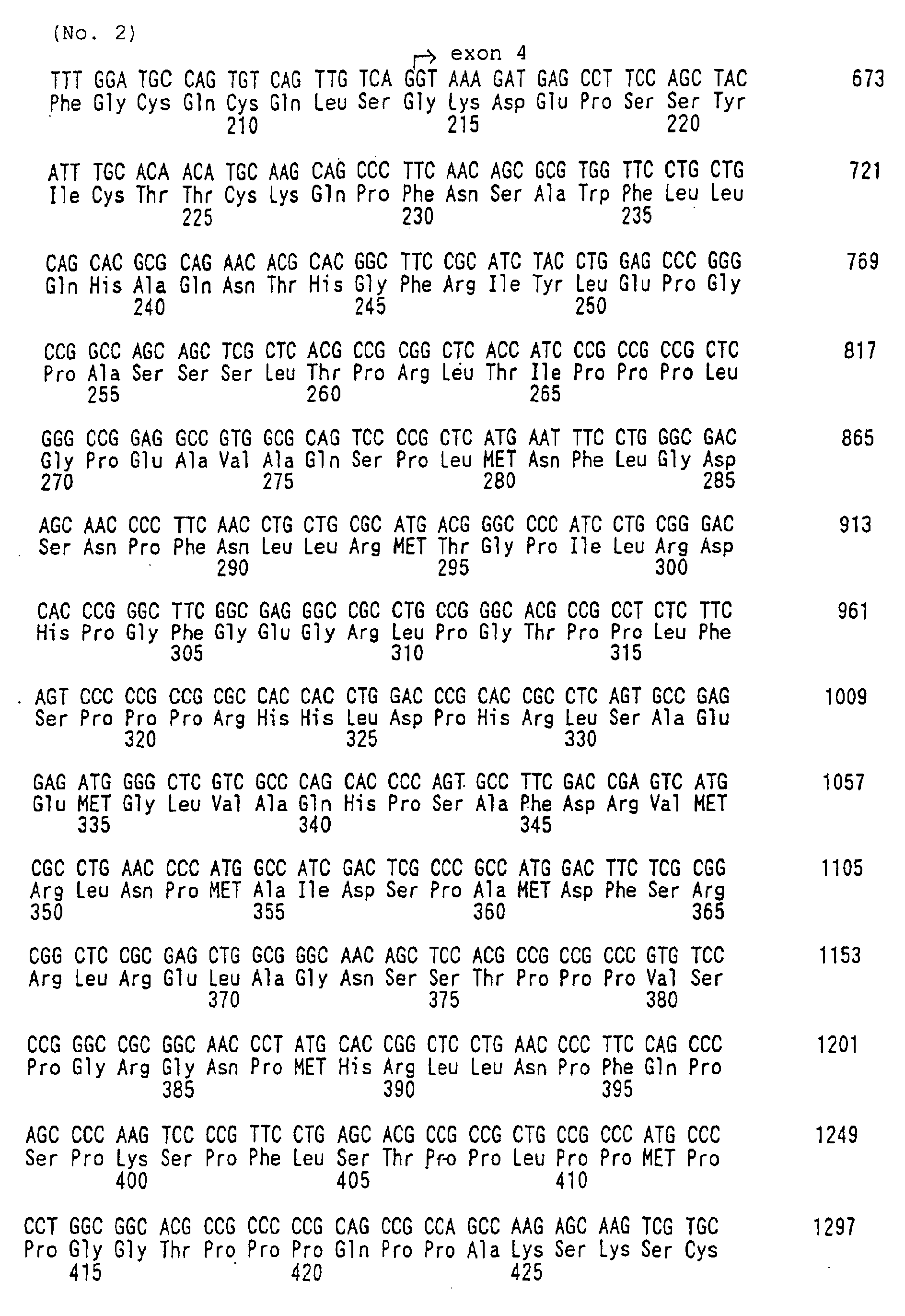

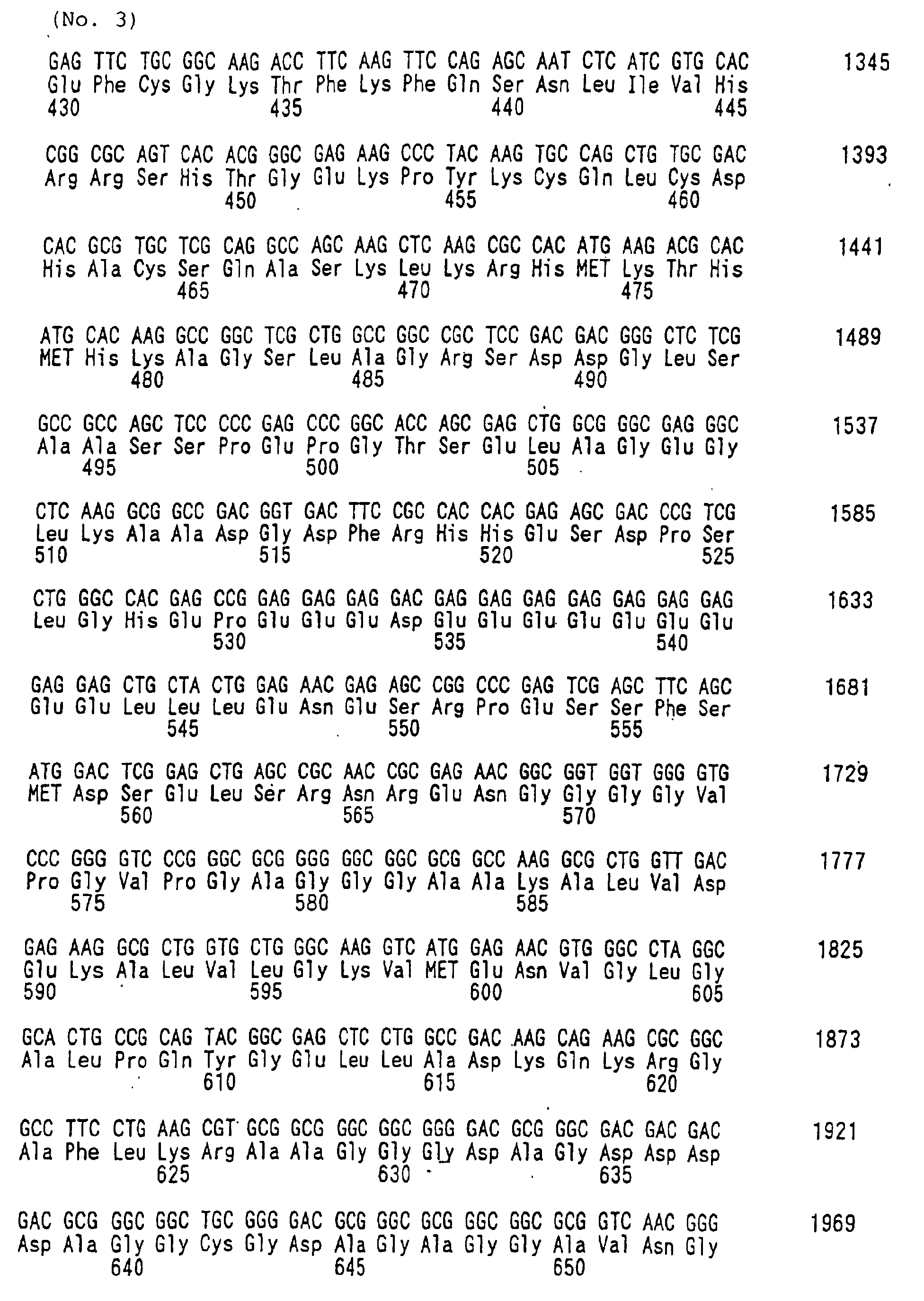

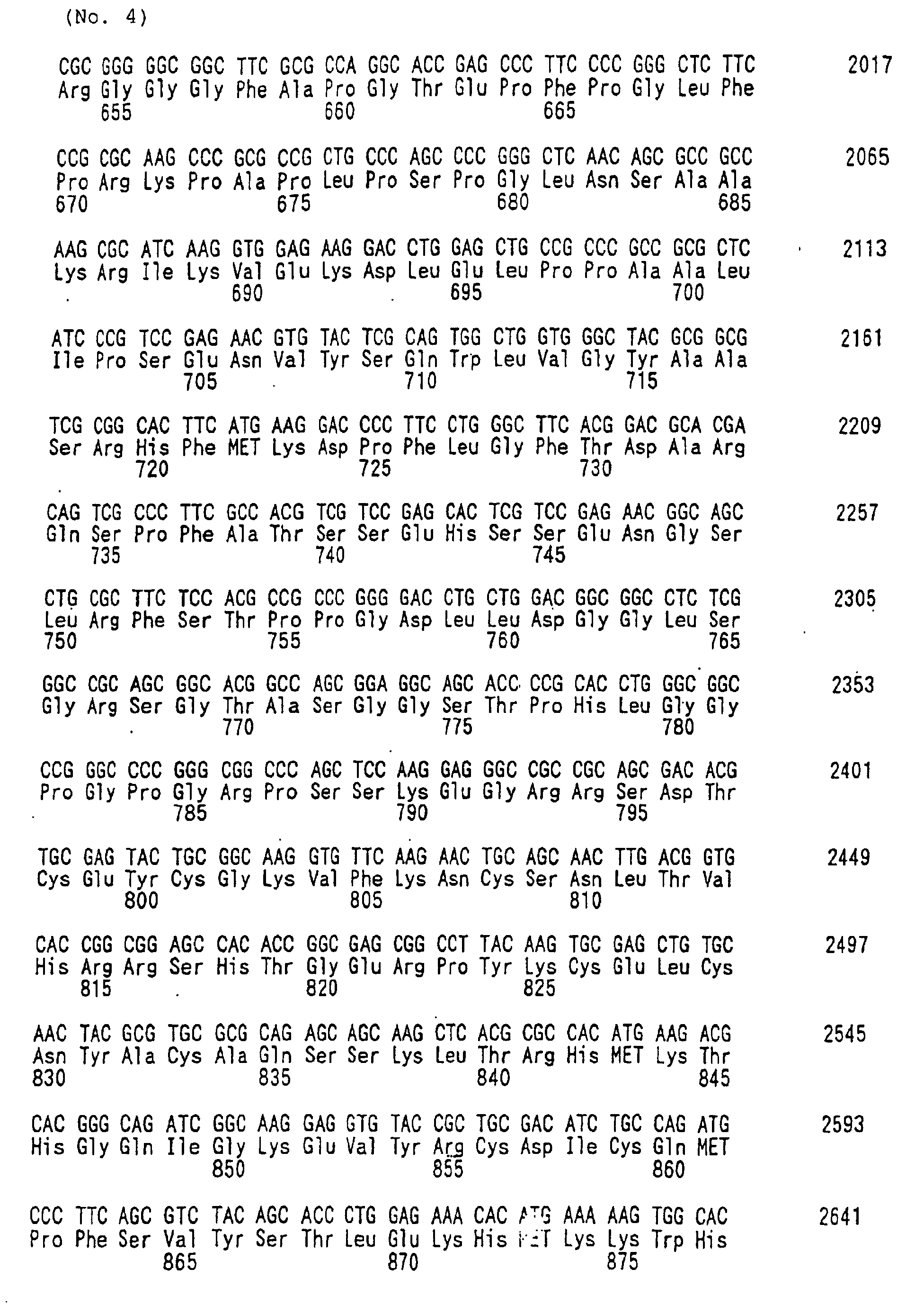

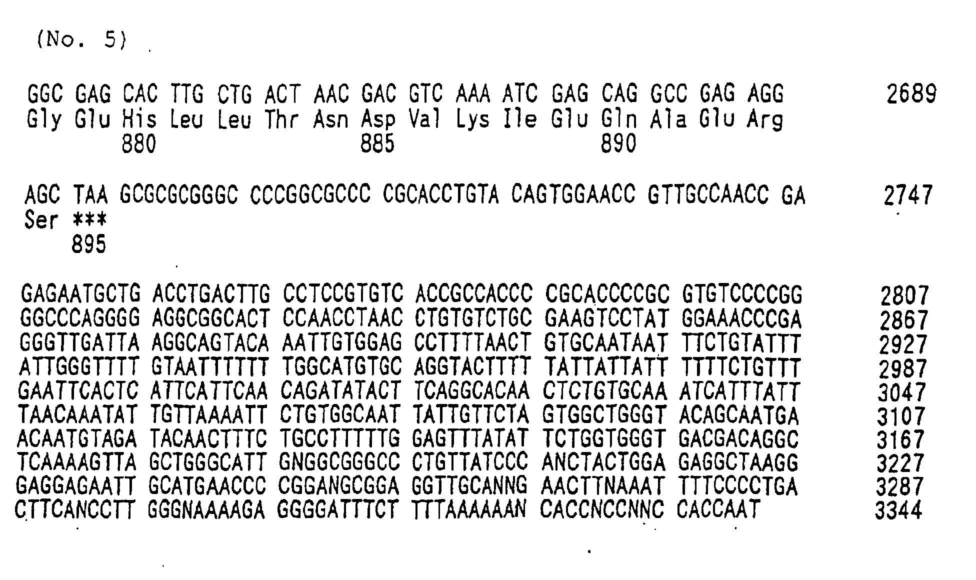

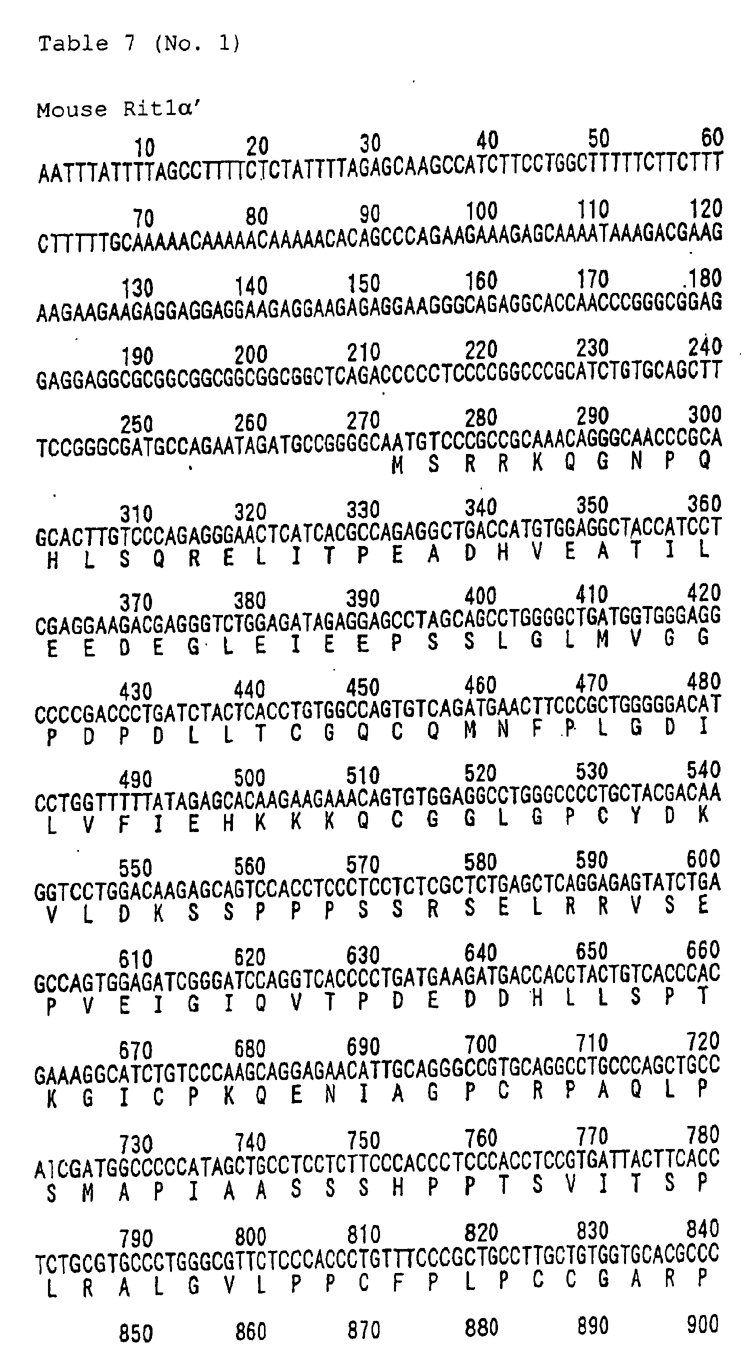

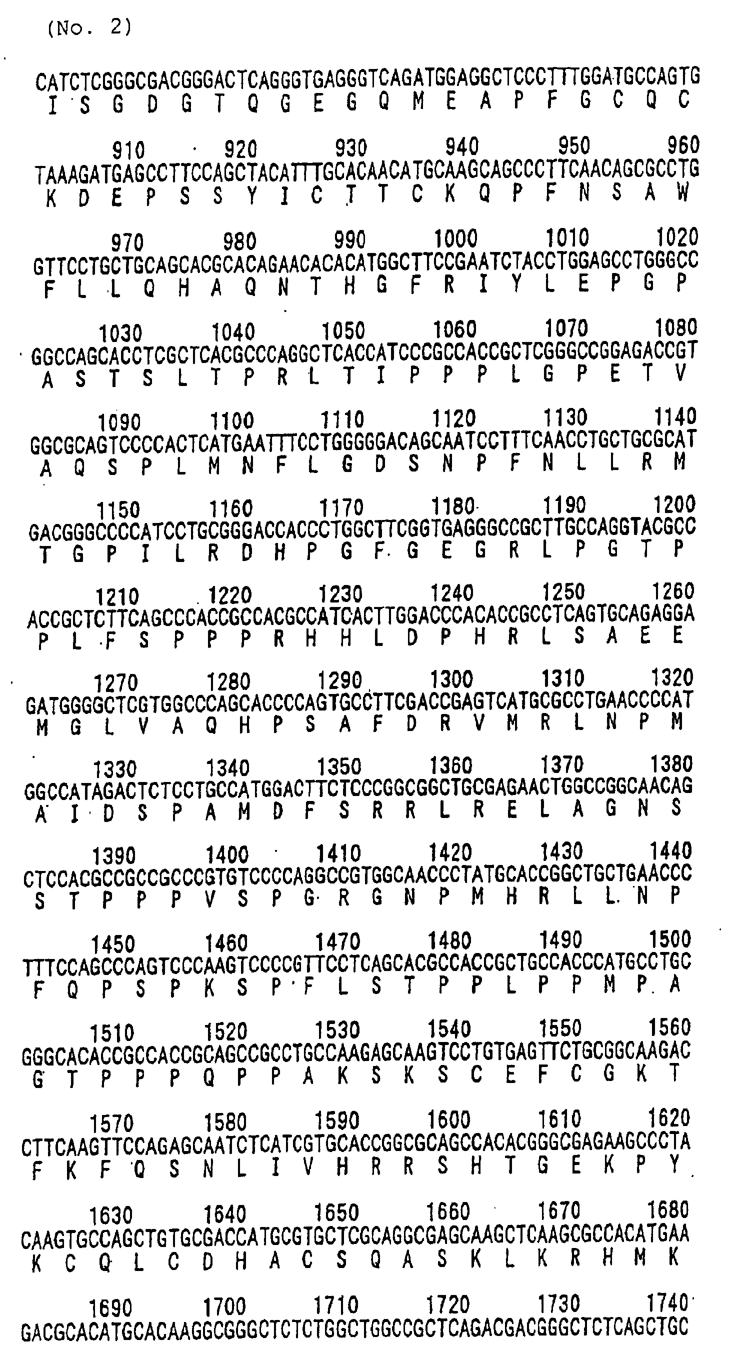

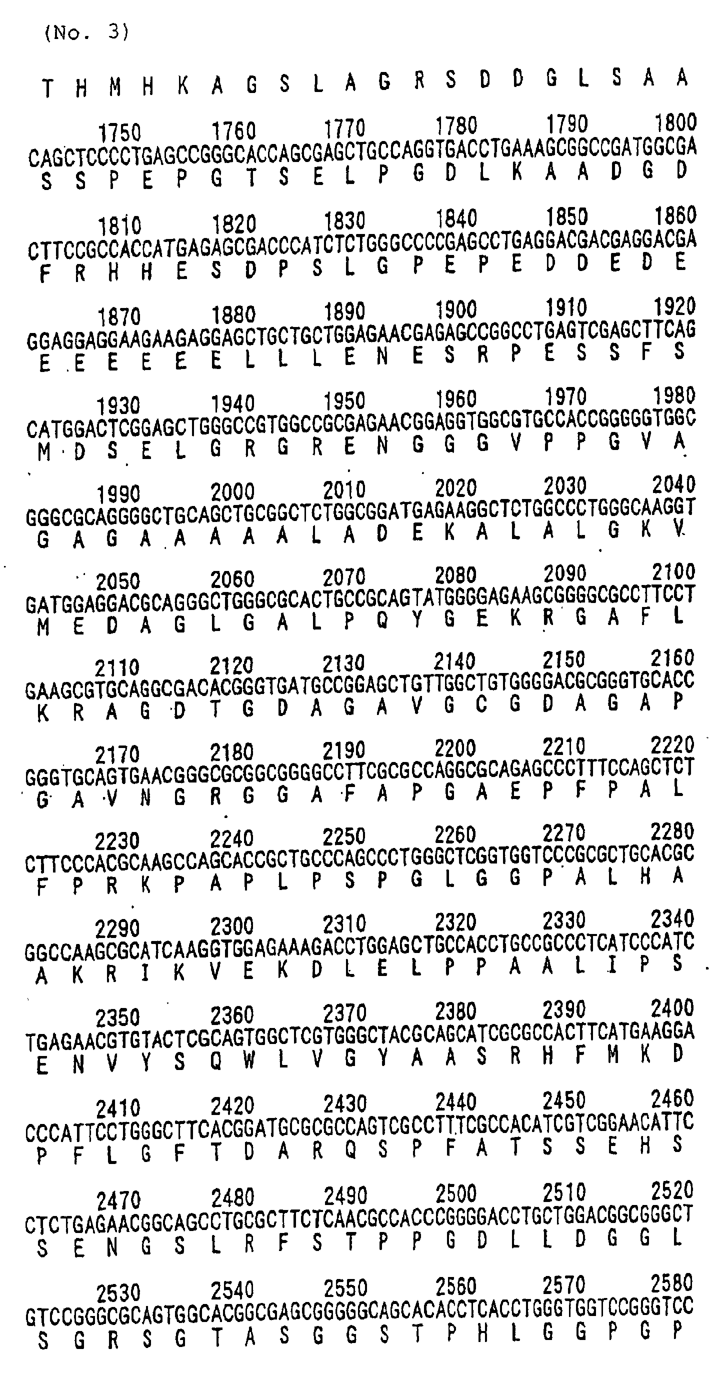

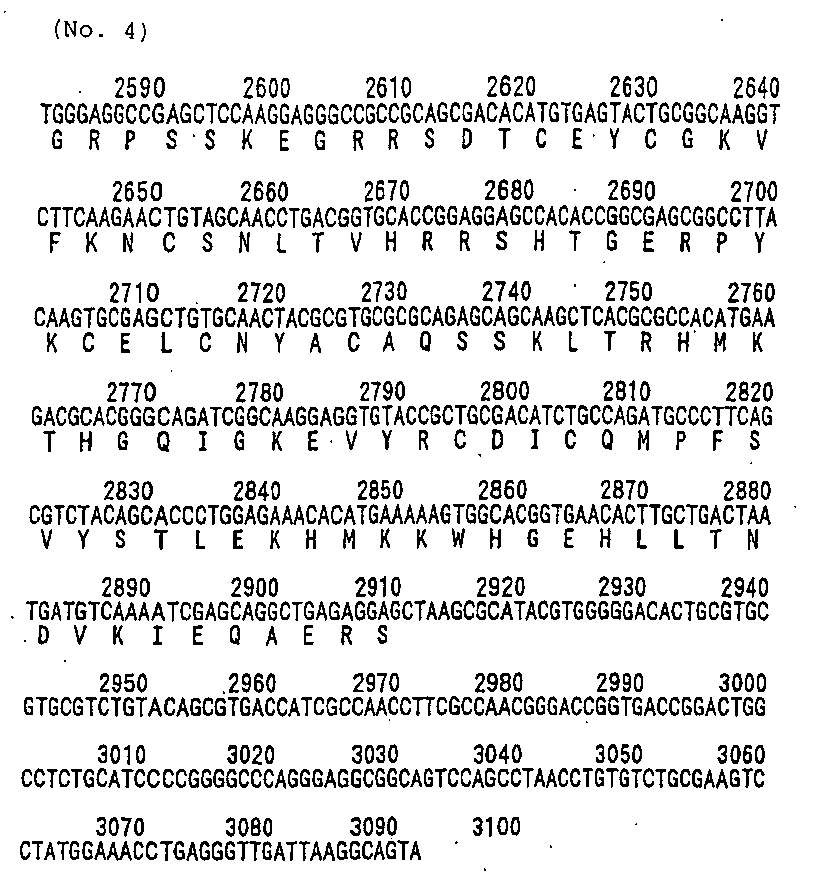

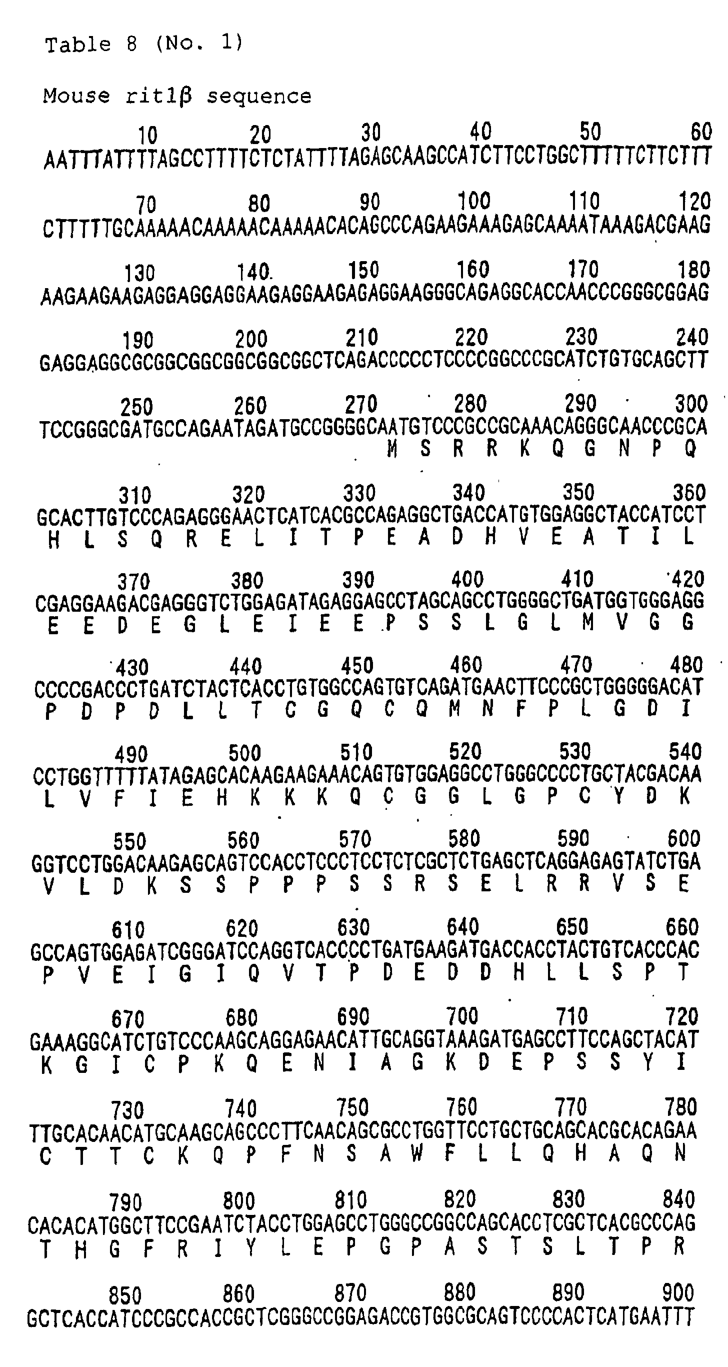

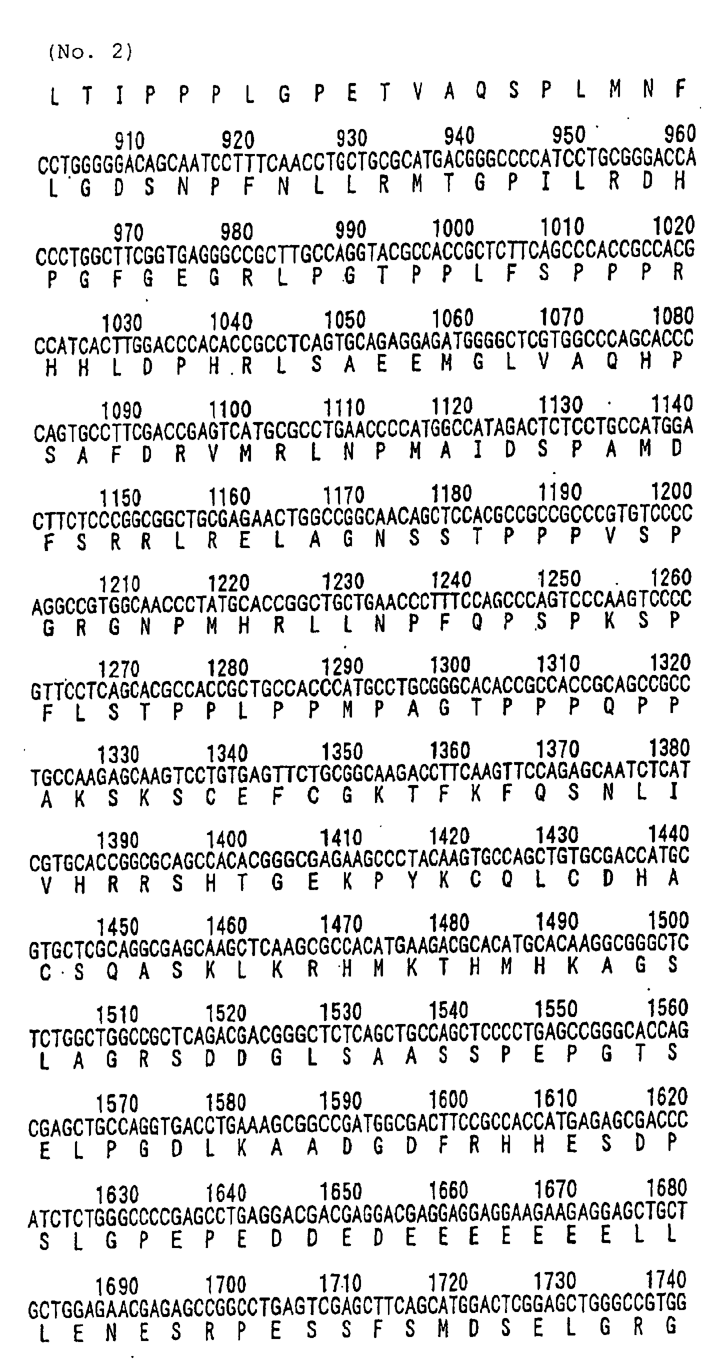

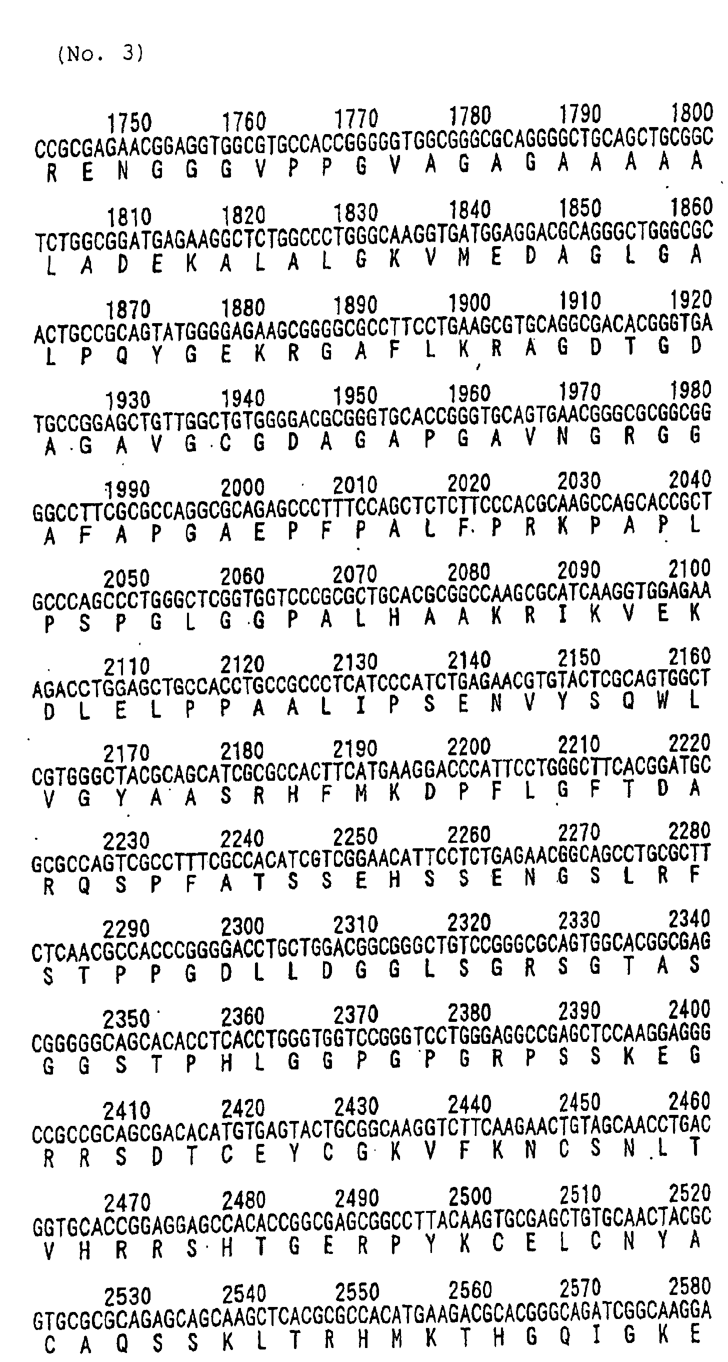

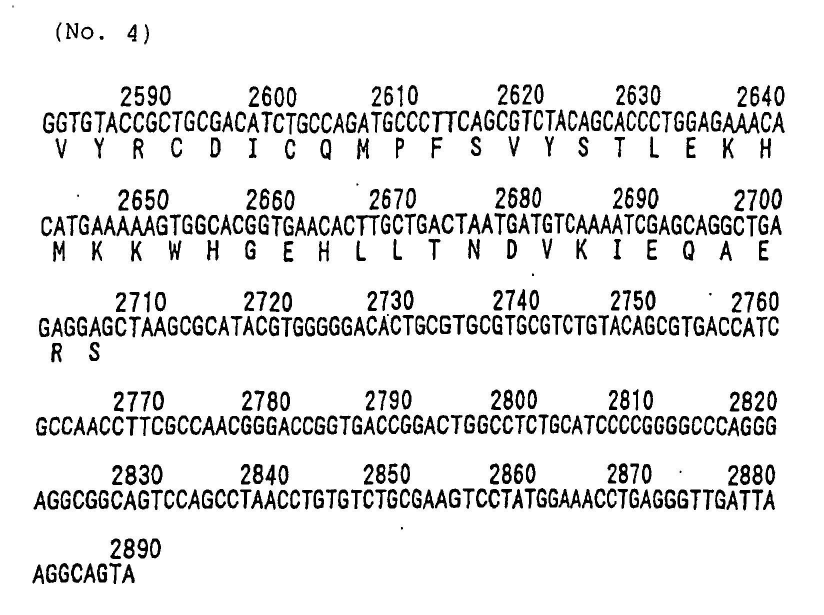

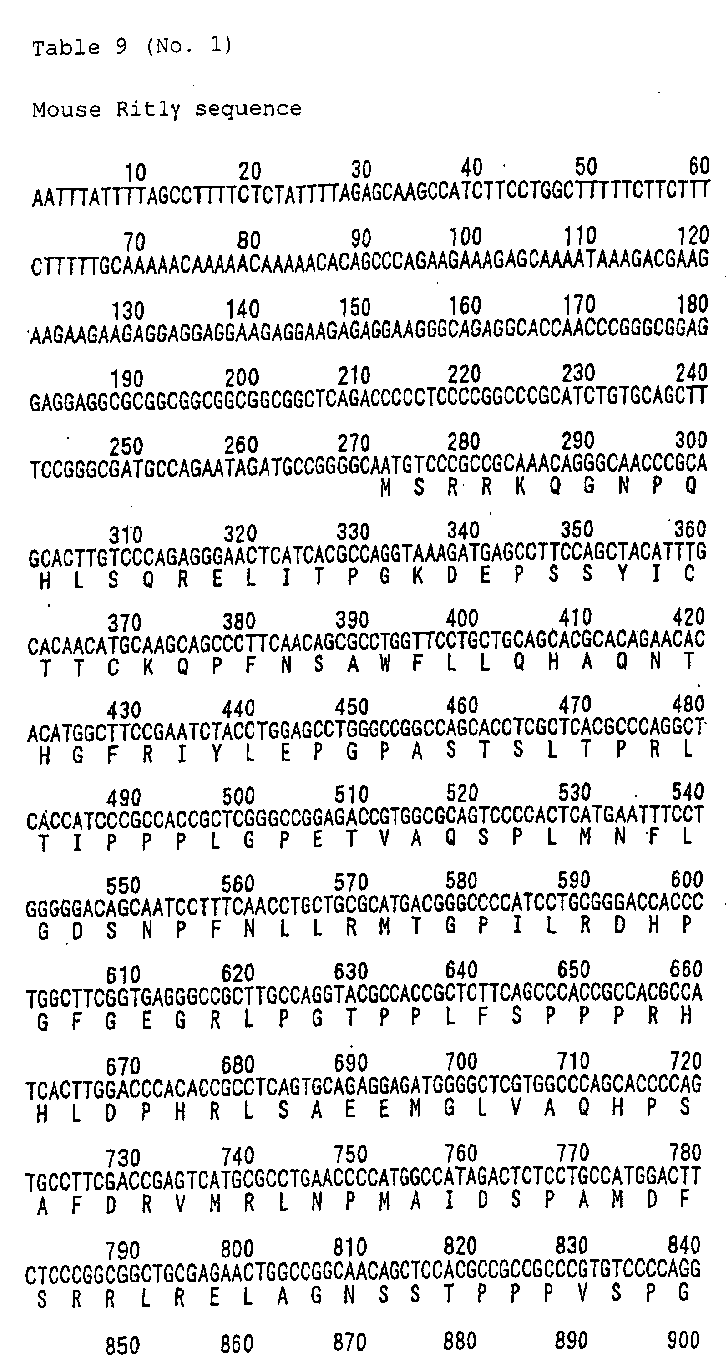

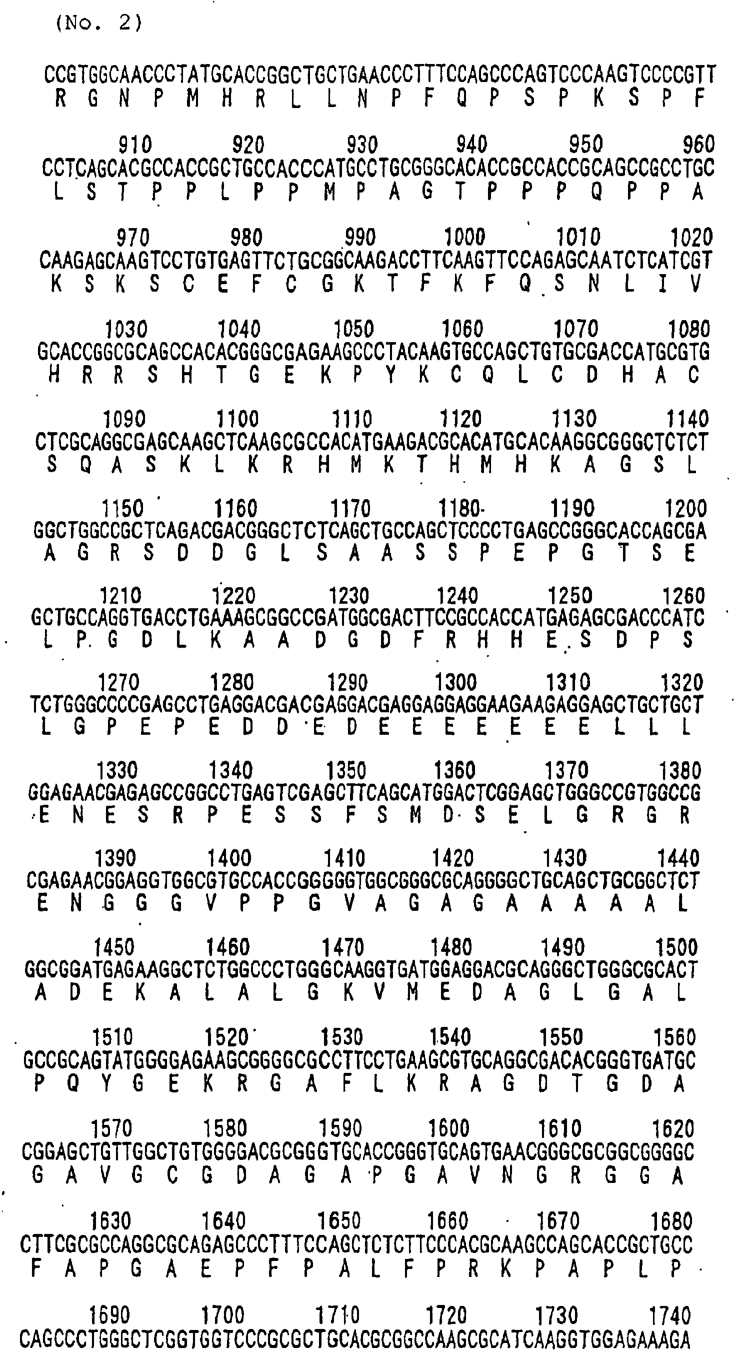

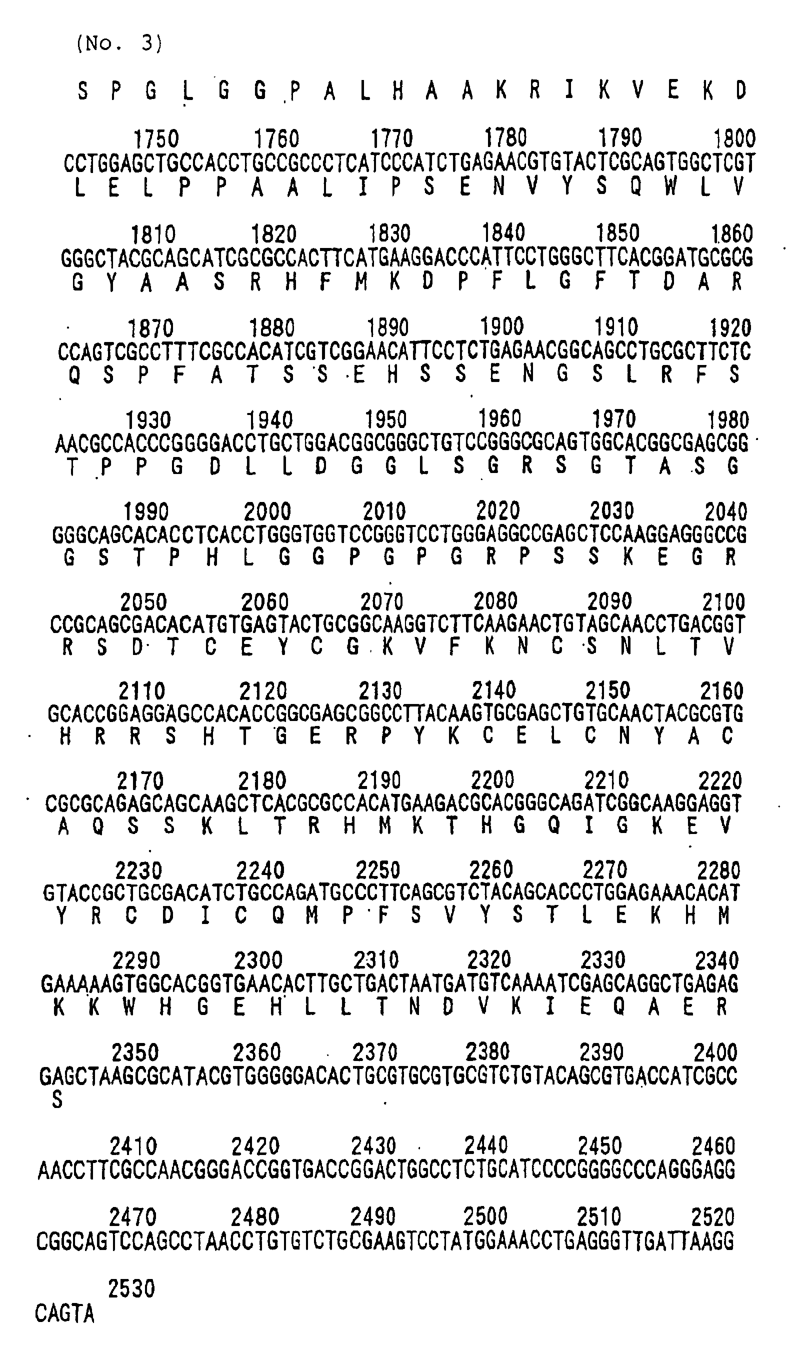

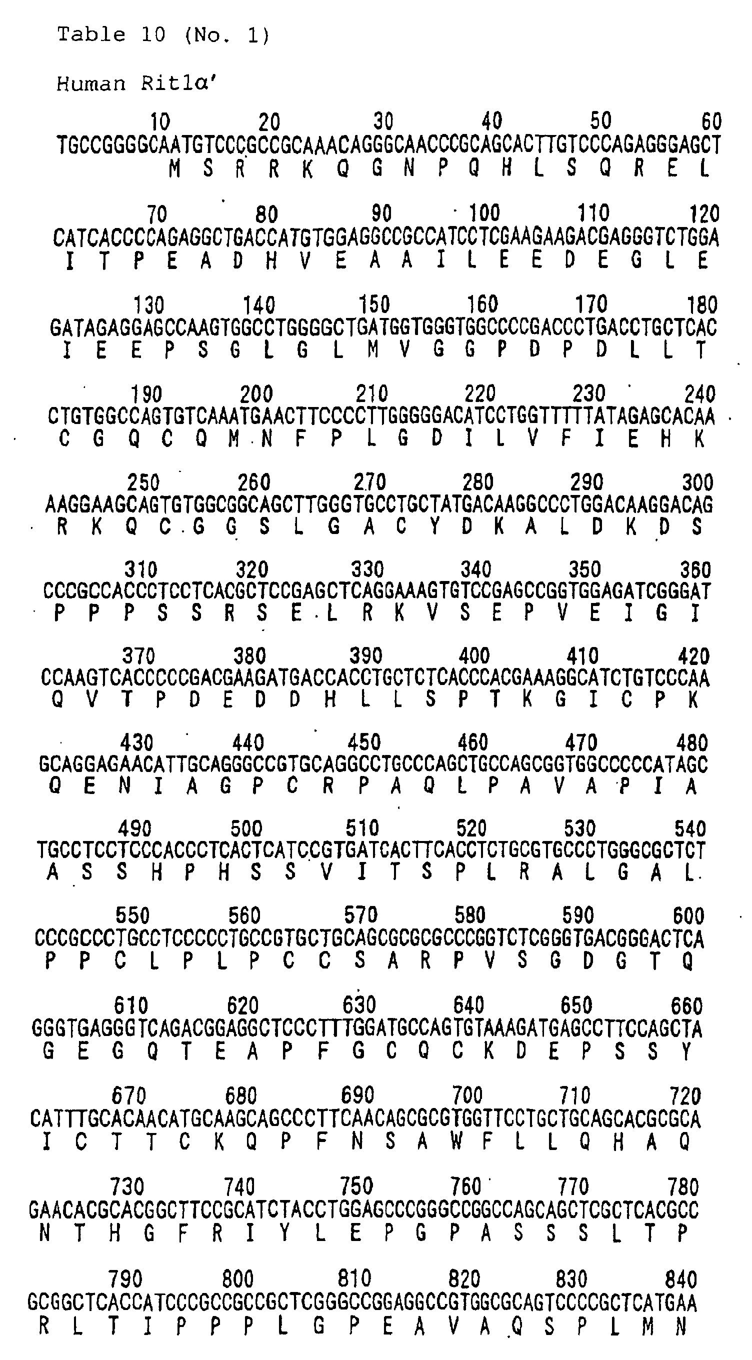

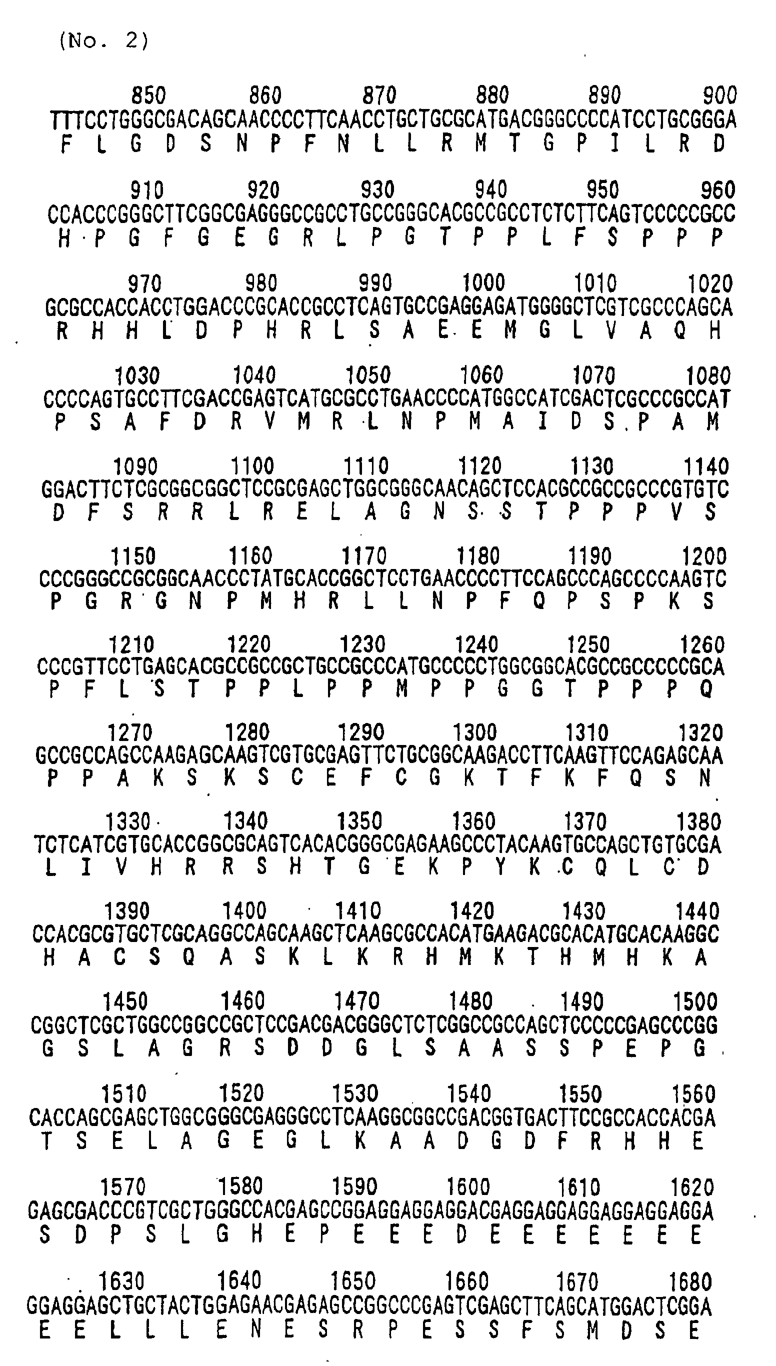

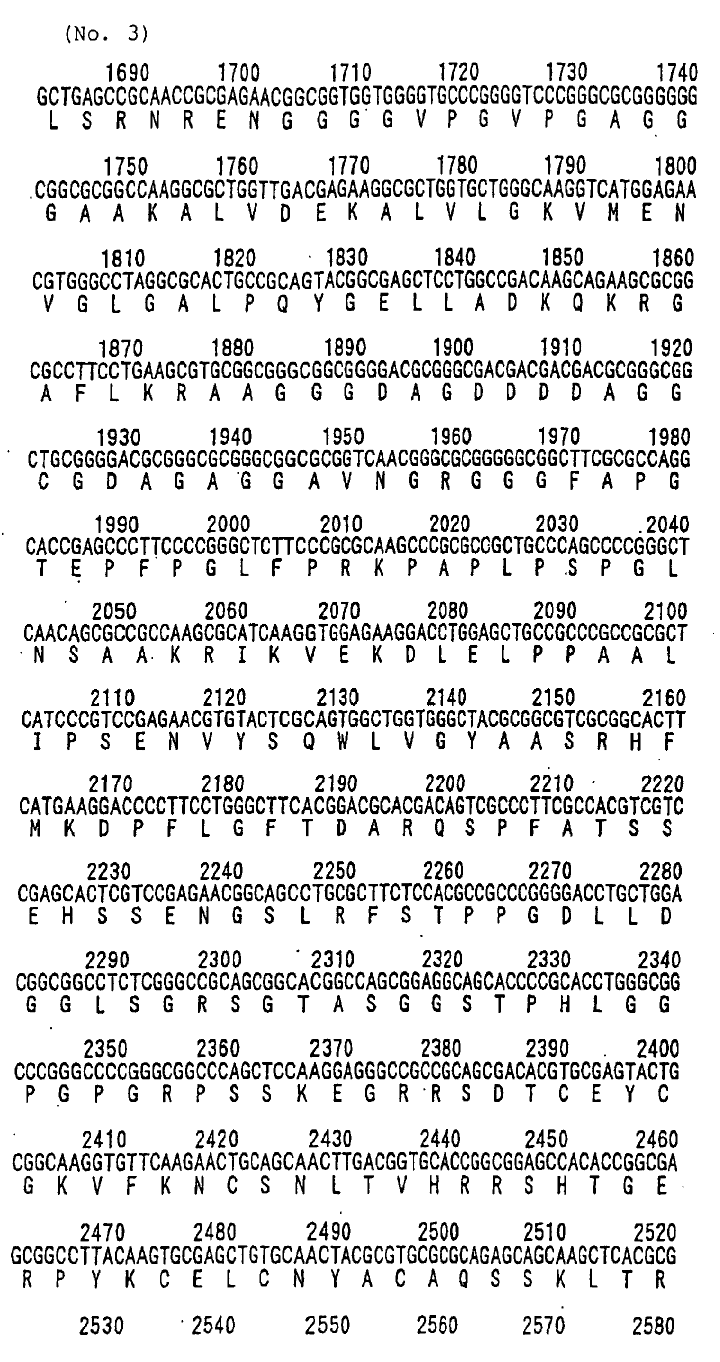

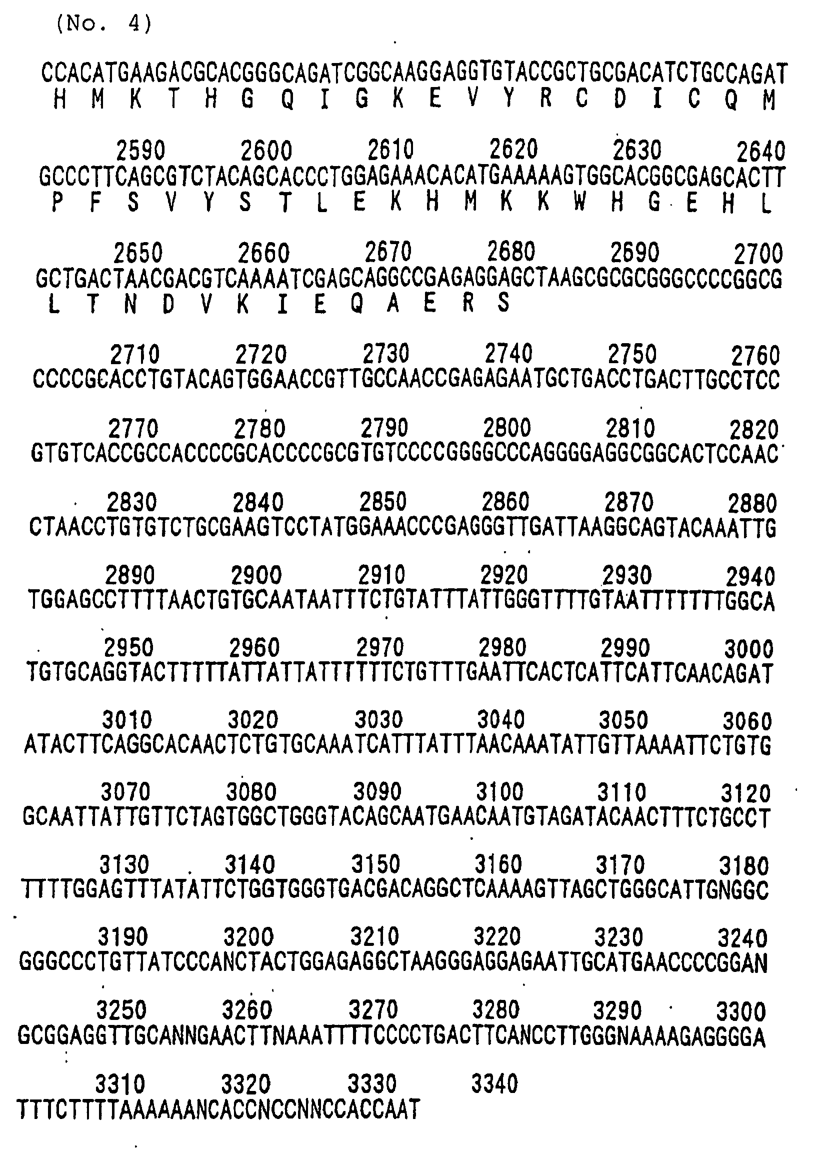

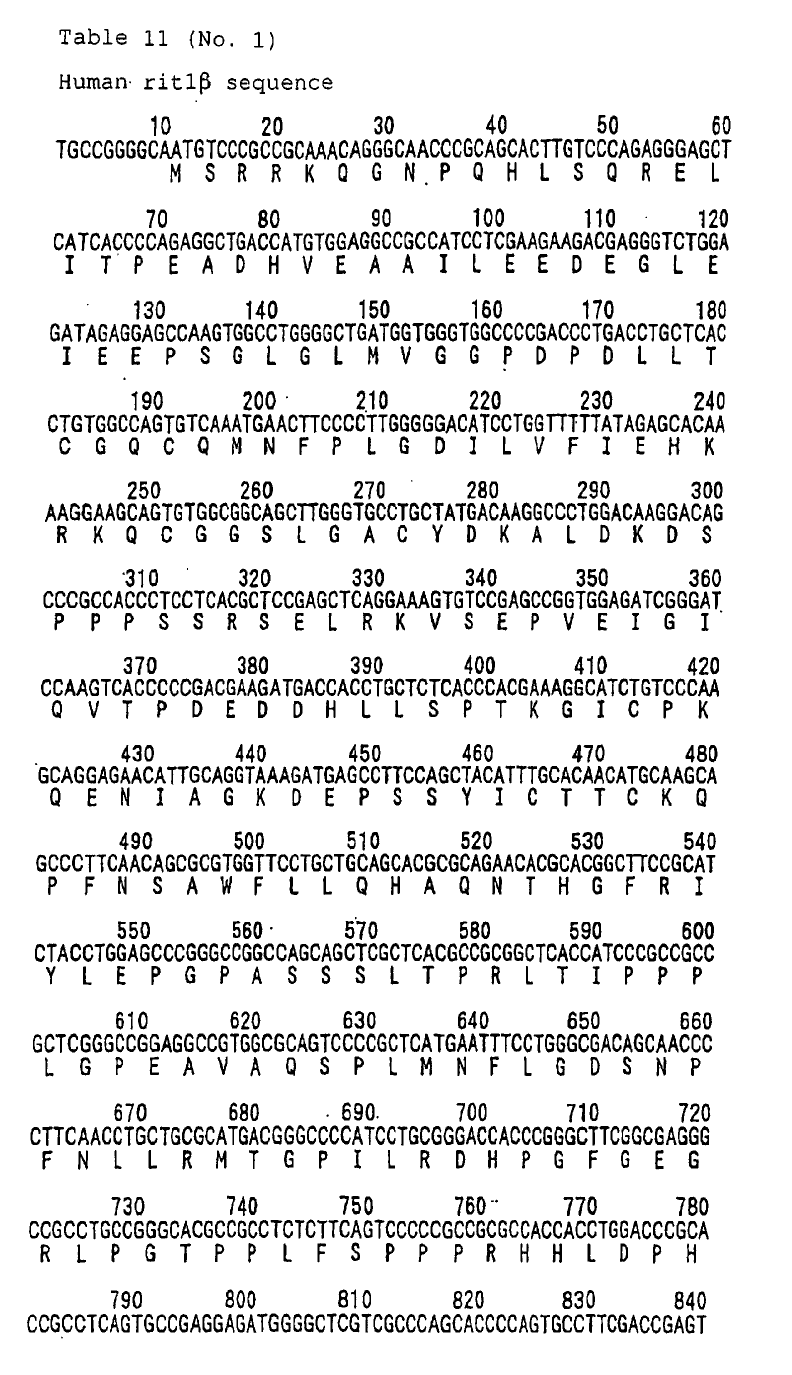

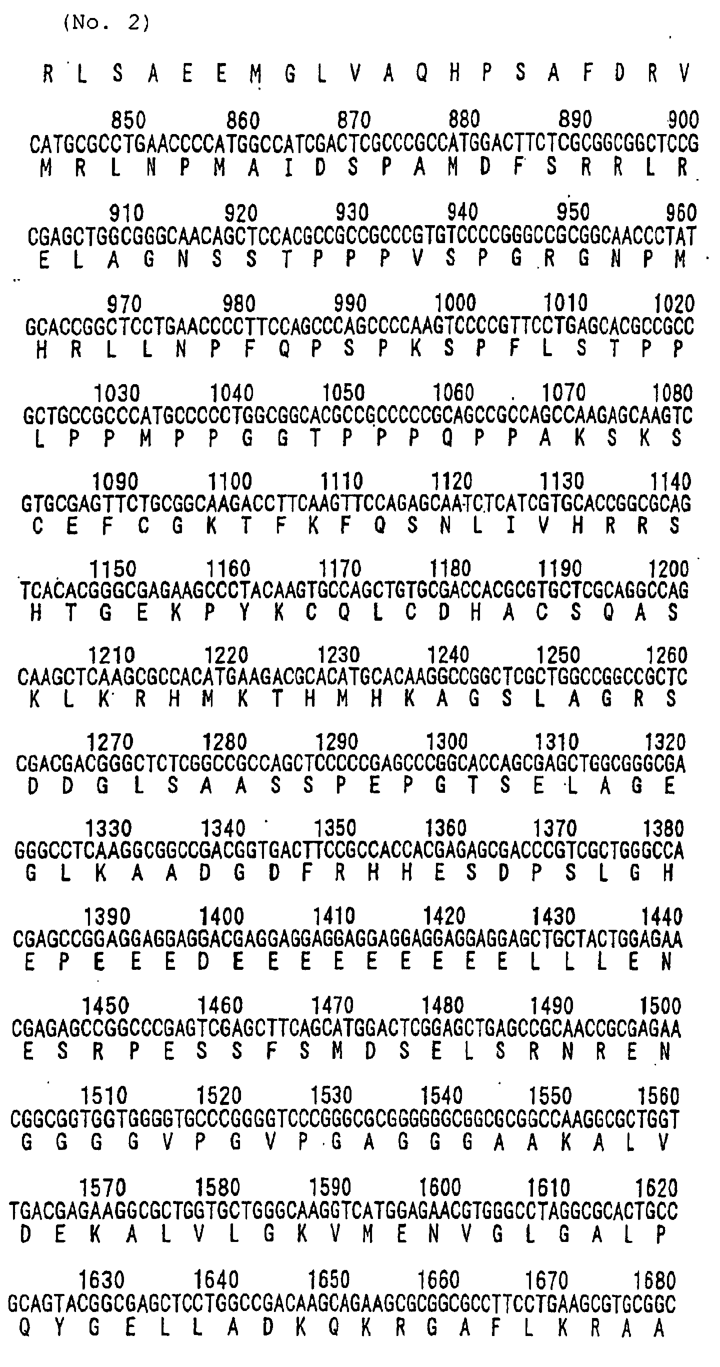

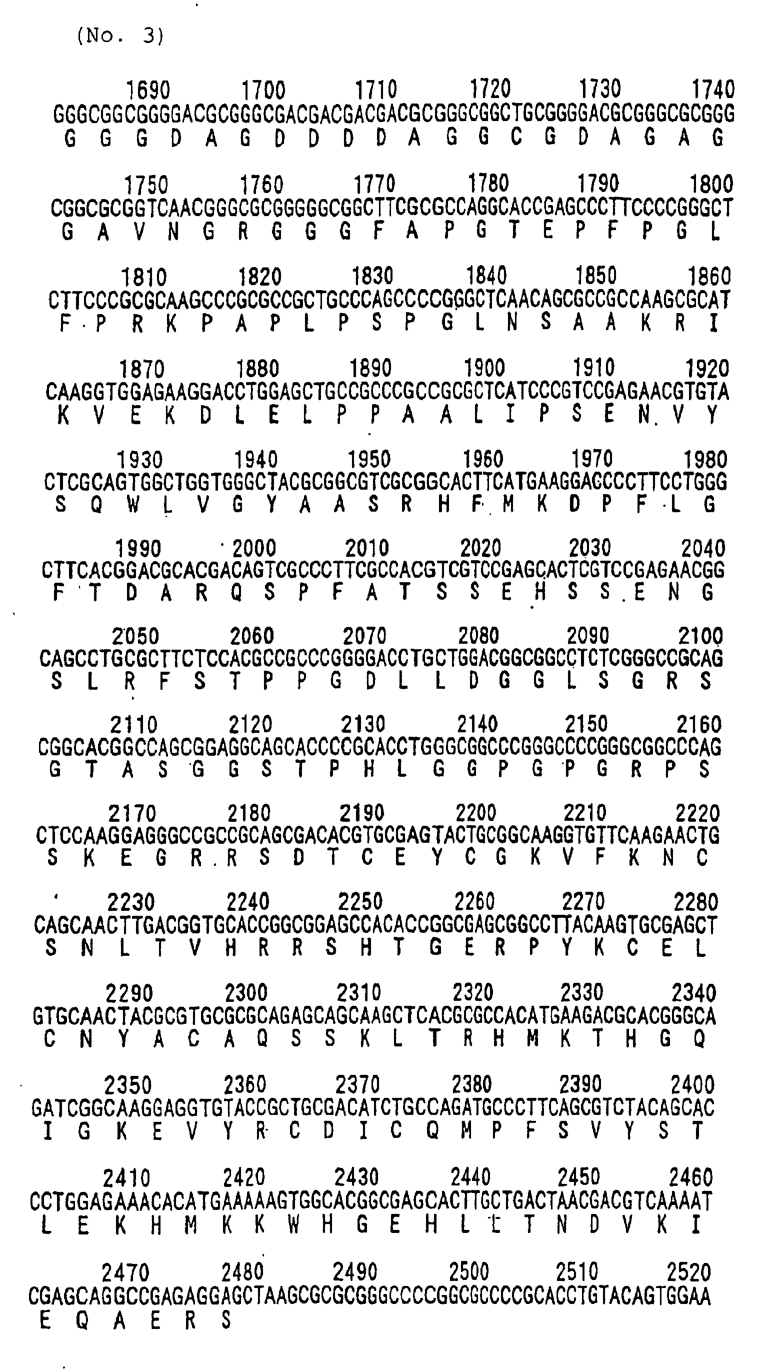

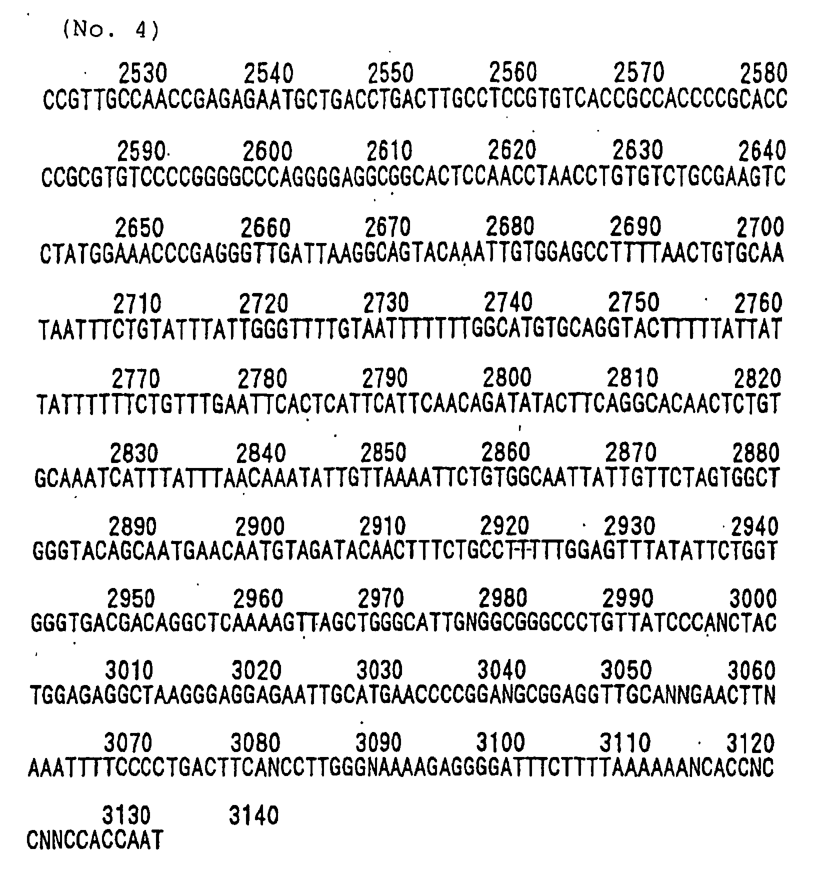

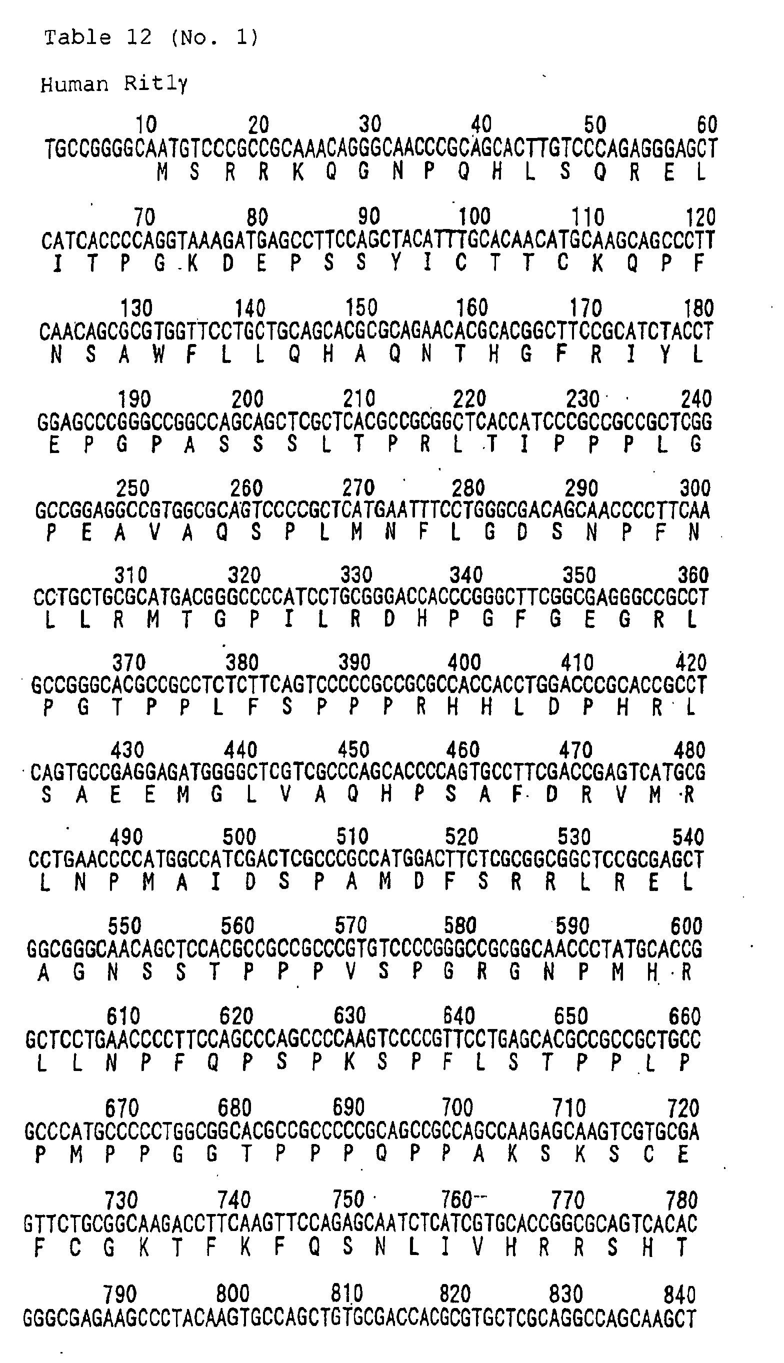

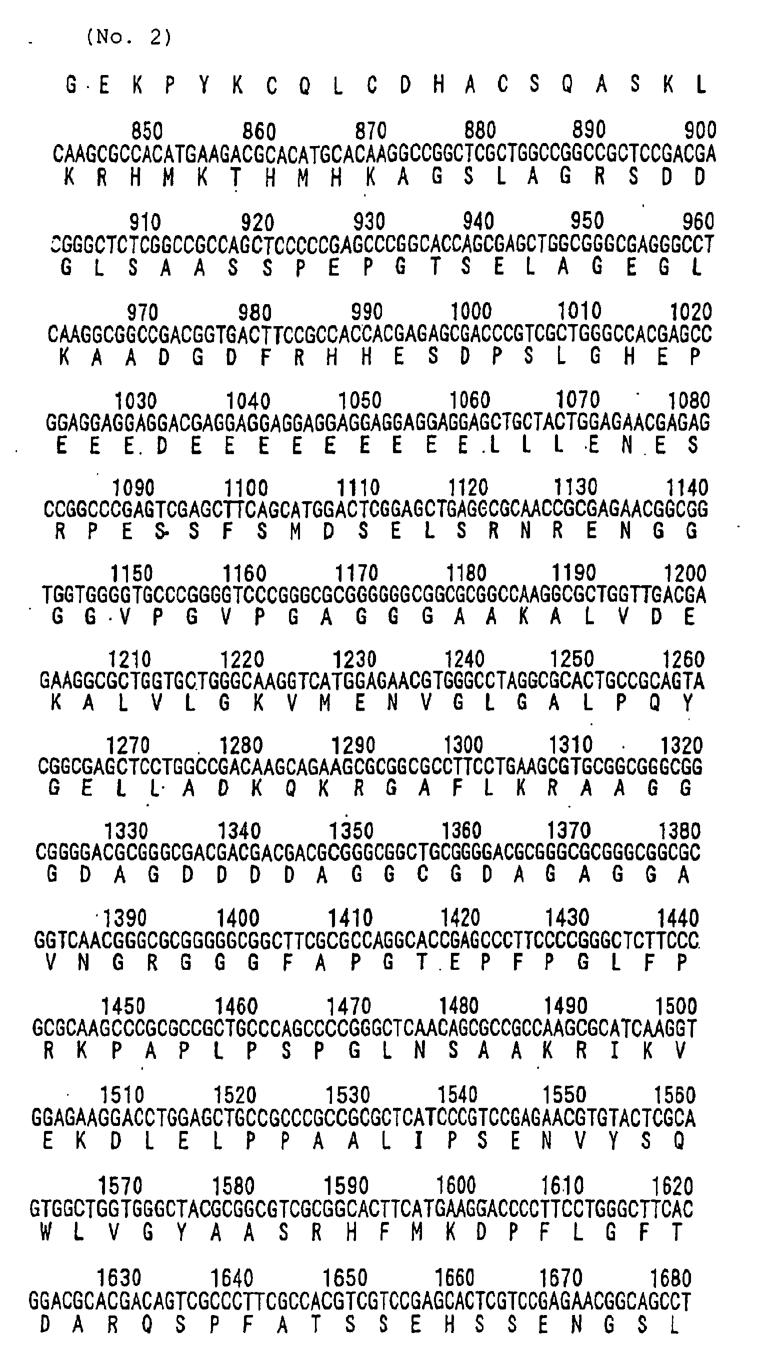

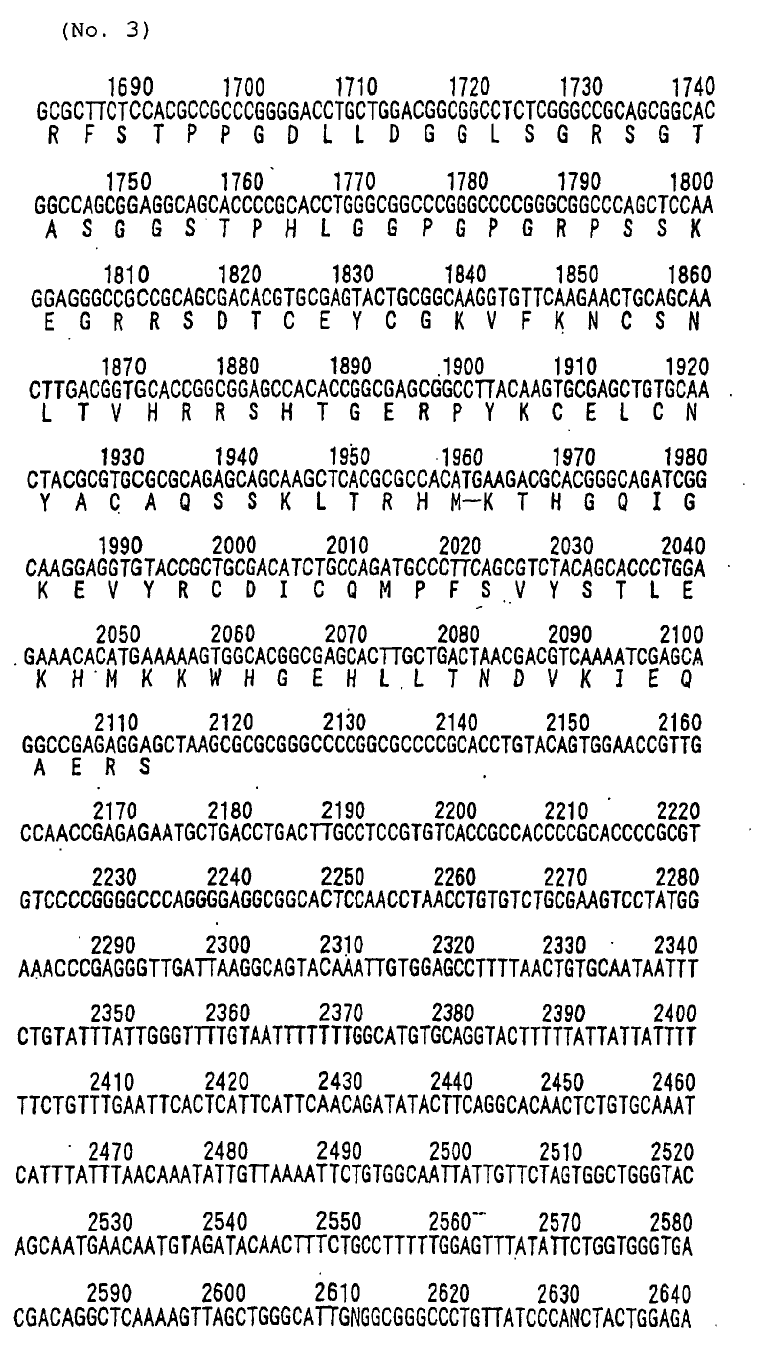

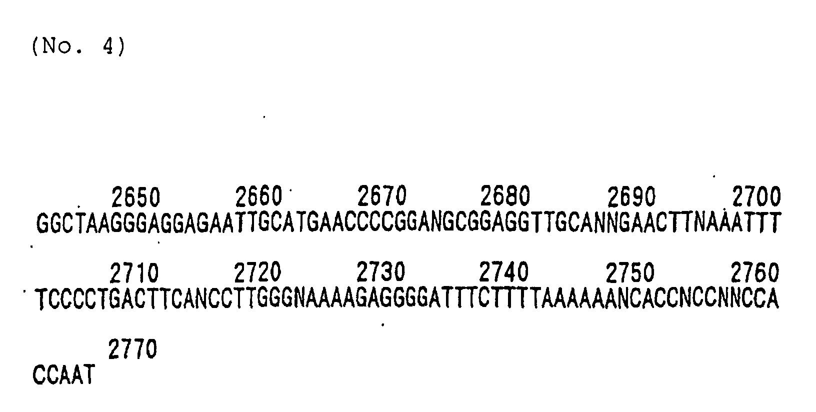

### INDUSTRIAL APPLICABILITY

This invention provides a novel tumor suppressor gene and a novel polypeptide which is an amplification product of the tumor suppressor gene. These gene and polypeptide can be used in diagnosing cancer, in estimating the cancer risk, and in the gene therapy.

## Claims

1. A novel polypeptide comprising a zinc finger structure of 2-3 type.

2. The novel polypeptide according to claim 1 wherein said polypeptide has tumor-suppressing activity.

3. The novel polypeptide according to claim 1 or 2 wherein said polypeptide comprises amino acid Nos. 1 to 884 or 47 to 884 in Table 1 (Sequence Listing, SEQ ID NO: 2).

4. The novel polypeptide according to claim 1 or 2 wherein said polypeptide comprises amino acid Nos. 1 to 894 or 47 to 894 of the amino acid sequence of Table 2 (Sequence Listing, SEQ ID NO: 4).

5. A polypeptide which is a part of the polypeptide of any one of claims 1 to 4.

6. A genomic DNA coding for the novel polypeptide of any one of claims 1 to 4.

7. A genomic DNA according to claim 6 wherein said DNA has at least one nucleotide sequence selected from nucleotide Nos. 2 to 332, 333 to 695, 696 to 911, and 912 to 2923 in the nucleotide sequence of Table 3 (Sequence Listing, SEQ ID NO: 1).

8. A genomic DNA according to claim 6 wherein said DNA has at least one nucleotide sequence selected from nucleotide Nos. 1 to 71, 72 to 437, 438 to 650, and 651 to 2692 in the nucleotide sequence of Table 4 (Sequence Listing, SEQ ID NO: 3).

9. A DNA fragment comprising a part of the genomic DNA of any one of claims 6 to 8.

10. A cDNA coding for the novel polypeptide of any one of claims 1 to 5.

11. The cDNA according to claim 10 wherein said cDNA comprises nucleotide Nos. 272 to 2923 or 410 to 2923 in the nucleotide sequence of Table 3 (Sequence Listing, SEQ ID NO: 1).

12. The cDNA according to claim 10 wherein said cDNA comprises nucleotide Nos. 113 to 3104 in the nucleotide sequence of Table 3 (Sequence Listing, SEQ ID NO: 1).

13. The cDNA according to claim 10 wherein said cDNA comprises nucleotide Nos. 11 to 2692 or 149 to 2692 in the nucleotide sequence of Table 4 (Sequence Listing, SEQ ID NO: 3).

14. A DNA fragment comprising a part of the cDNA of any one of claims 10 to 13.

15. A DNA fragment according to 14 wherein said cDNA part includes the nucleotide sequence coding for a zinc finger.

16. A DNA fragment comprising a nucleotide sequence which is a part of the sense strand of the cDNA of any one of claims 10 to 13.

17. A DNA fragment comprising a nucleotide sequence which is a part of the antisense strand of the cDNA of any one of claims 10 to 13.

18. A vector including the DNA of any one of claims 6 to 8.

19. A vector including the DNA of any one of claims 10 to 13.

20. A transformant produced by transformation using the vector of claim 19.

21. A novel antibody produced by administering the polypeptide of any one of claims 1 to 5 as an antigen.

22. The novel antibody according to claim 21 which recognizes the polypeptide of any one of claims 1 to 5.

23. A method for diagnosing cancer **characterized in that**, in said method, a test is conducted to determine whether or not the novel polypeptide of any one of claims 1 to 4 is normally expressed in the cell, or whether the DNA of any one of claims 6 to 13 has experienced any mutation or polymorphism.

24. A method for estimating cancer risk **characterized in that**, in said method, a test is conducted to determine whether or not the novel polypeptide of any one of claims 1 to 4 is normally expressed in the cell, or whether the DNA of any one of claims 6 to 13 has experienced any mutation or polymorphism.

25. The method according to claim 23 or 24 comprising at least the steps of:
(a) extracting DNA from a cell or a tissue;
(b) producing a primer comprising at least 15 nucleotides which includes the sequence of an arbitrary part of the DNA of any one of claims 6 to 13;
(c) conducting PCR for the DNA of (a) using the primer of (b); and
(d) analyzing the result of (c).

26. The method according to claim 23 or 24 comprising at least the steps of:
(a) extracting mRNA from a cell or a tissue;
(b) producing a primer comprising at least 15 nucleotides which includes the sequence of an arbitrary part of the DNA of any one of claims 6 to 13;
(c) conducting RT-PCR for the DNA of (a) using the primer of (b); and
(d) analyzing the result of (c).

27. A medical composition for cancer treatment **characterized in that** said composition contains the DNA of any one of claims 6 to 8.

28. A medical composition for cancer treatment **characterized in that** said composition contains the DNA of any one of claims 10 to 13.

29. A polypeptide having the amino acid sequence of Sequence Listing, SEQ ID NO: 6.

30. A polypeptide having the amino acid sequence of Sequence Listing, SEQ ID NO: 8.

31. A polypeptide having the amino acid sequence of Sequence Listing, SEQ ID NO: 10.

32. A polypeptide having the amino acid sequence of Sequence Listing, SEQ ID NO: 12.

33. A polypeptide having the amino acid sequence of Sequence Listing, SEQ ID NO: 14.

34. A polypeptide having the amino acid sequence of Sequence Listing, SEQ ID NO: 16.

35. A DNA having the nucleotide sequence of Sequence Listing, SEQ ID NO: 5.

36. A DNA having the nucleotide sequence of Sequence Listing, SEQ ID NO: 7.

37. A DNA having the nucleotide sequence of Sequence Listing, SEQ ID NO: 9.

38. A DNA having the nucleotide sequence of Sequence Listing, SEQ ID NO: 11.

39. A DNA having the nucleotide sequence of Sequence Listing, SEQ ID NO: 13.

40. A DNA having the nucleotide sequence of Sequence Listing, SEQ ID NO: 15.
